(19) Europäisches Patentamt
European Patent Office
Office européen des brevets

(11) EP 2 974 744 A1

(12) EUROPEAN PATENT APPLICATION

(43) Date of publication:
**20.01.2016 Bulletin 2016/03**

(21) Application number: **14177230.1**

(22) Date of filing: **16.07.2014**

(51) Int Cl.:
***A61K 47/48*** (2006.01) ***A61K 47/32*** (2006.01)
***C07D 225/08*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Stichting Katholieke Universiteit**
**6525 EZ Nijmegen (NL)**

(72) Inventors:
- **van Delft, Floris Louis**
  **Nijmegen (NL)**
- **Fatunsin, Olumide Foluso**
  **Nijmegen (NL)**
- **Borrmann, Annika**
  **Nijmegen (NL)**
- **van Hest, Jan Cornelis Maria**
  **Nijmegen (NL)**
- **Löwik, Dennis Wilhelmus Petrus Maria**
  **Nijmegen (NL)**
- **Jonker, Anika Maartje**
  **Nijmegen (NL)**

(74) Representative: **Ellens, Andries**
**Ellens & van Essen**
**Agro Business Park 20**
**6708 PW Wageningen (NL)**

(54) **Strain-promoted oxidation controlled cycloaddition with high reaction rate**

(57) The invention provides a the use of a (hetero)cycloalkyne and an oxo-group containing (hetero)1,3-diene for coupling a first system (A) of interest to a second system (A') of interest, wherein a (hetero)cycloalkyne is created in one of the first system (A) of interest and the second system (A') of interest, and wherein an oxo-group containing (hetero)1,3-diene is created in the other system of interest, and allowing the (hetero)cycloalkyne and the oxo-group containing (hetero)1,3-diene to react to form an A-A' conjugate.

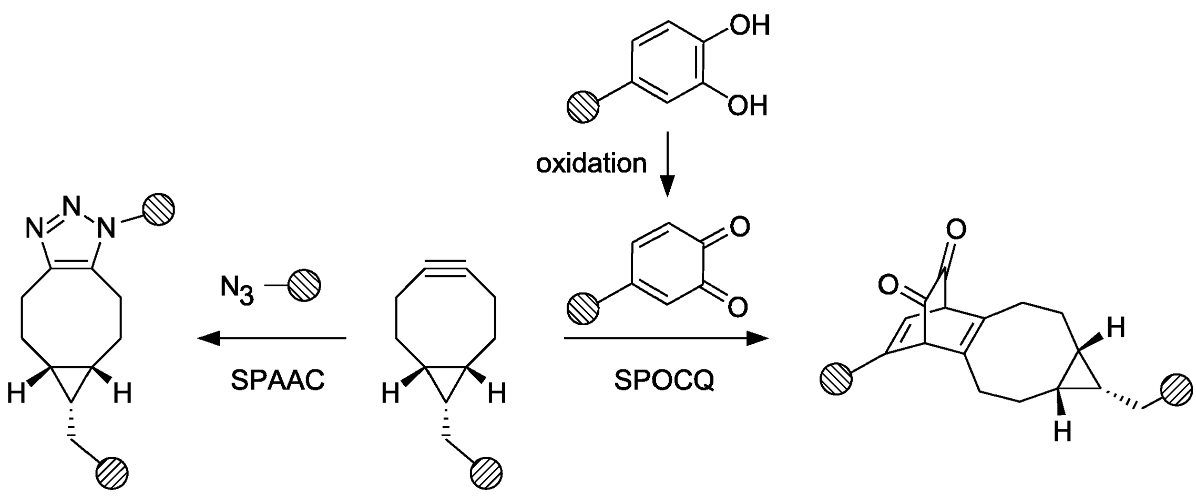


FIG. 1

EP 2 974 744 A1

## Description

FIELD OF THE INVENTION

The invention relates to a method for making a conjugate, such as e.g. a protein-label conjugate, as well as to such conjugates per se. Further, the invention also relates to the use of (hetero)cycloalkyne in orthogonal (bio)chemistry.

BACKGROUND OF THE INVENTION

As amongst others described by Bertozzi et al. in WO 2006/050262, selective chemical reactions that are orthogonal to the diverse functionality of biological systems are now recognized as important tools in chemical biology. As relative newcomers to the repertoire of synthetic chemistry, these bioorthogonal reactions have inspired new strategies for compound library synthesis, protein engineering, functional proteomics, and chemical remodeling of cell surfaces. The azide has secured a prominent role as a unique chemical handle for bioconjugation. The Staudinger ligation has been used with phosphines to tag azidosugars metabolically introduced into cellular glycoconjugates. The Staudinger ligation can be performed in living animals without physiological harm; nevertheless, the Staudinger reaction is not without liabilities. The requisite phosphines are susceptible to air oxidation and their optimization for improved water solubility and increased reaction rate has proven to be synthetically challenging. The azide group has an alternative mode of bioorthogonal reactivity: the [3+2] cycloaddition with alkynes. In its classic form, this reaction has limited applicability in biological systems due to the requirement of elevated temperatures (or pressures) for reasonable reaction rates. This obstacle was surmounted with the development of a copper(I)-catalyzed version, termed "click chemistry," that proceeds readily at physiological temperatures and in richly functionalized biological environs. This discovery has enabled the selective modification of virus particles, nucleic acids, and proteins from complex tissue lysates. Unfortunately, the mandatory copper catalyst is toxic to both bacterial and mammalian cells, thus precluding applications wherein the cells must remain viable. Catalyst-free Huisgen cycloadditions of alkynes activated by electron-withdrawing substituents have been reported to occur at ambient temperatures. However, these compounds undergo Michael reaction with biological nucleophiles.

SUMMARY OF THE INVENTION

Besides the use of click chemistry in the area of chemical biology and bioconjugation, this versatile toolbox has also found widespread application in the polymer and materials community. Click chemistry has been used for the construction of block copolymers, polymer modification reactions and the build-up of polymer networks such as hydrogels. Also many immobilization strategies employ click chemistry because of its reaction rate, effectiveness and selectivity. There is however a great need for click reactions which are both fast and activatable. For example, many hydrogel applications are directed to in vivo use, which implies that the hydrogels have to be injected. A shortcoming of current materials is their too slow network formation process, which can be overcome by the development of a fast, activatable coupling reaction. Furthermore, spatiotemporal control over fast reactions will find its application in the area of 3D printed materials. Also self-healing materials, which can be restored in their original properties by an activatable reaction will benefit from the herein proposed development. With respect to immobilization, patterning requires an activation step to allow positioning of polymers or biomolecules with a high level of spatial resolution.

The modification of proteins is key to many (bio)technological and diagnostic applications, including surface immobilization and conjugation of proteins to specific reporter molecules such as fluorophores, radioisotope labels or chelators. A well-known example of tailoring protein properties for clinical application involves the covalent attachment of a polyethylene glycol chain (PEGylation) to increase the circulation half-life of a protein. Proteins have also been conjugated to drugs and have been used as drug-delivery systems for therapy or diagnosis purposes. In this respect, a high level of control over the site of modification is particularly relevant, since a structural change can have direct impact on protein function.

Amide bond formation with lysine side-chains or thiol-maleimide chemistry for the reaction with cysteines generally lack site-selectivity, because either amino acid is typically present multiple times in a given protein. In contrast, ketones and aldehydes form a class of non-proteinogenic reactive groups, which enable conjugation under mild conditions that prevent protein denaturation. Selective labeling of proteins can also be achieved using Staudinger ligation, Cu(I) catalyzed alkyne-azide cycloaddition (CuAAC), or strain-promoted cycloaddition of cyclooctynes with 1,3-dipoles such as azides (SPAAC), nitrones (SPANC), nitrile oxides (SPANOC), diazo compounds or sydnones. Many different technologies have been developed to site-specifically introduce one of these chemical handles into proteins involving genetic, enzymatic and chemical approaches. The strain-promoted inverse electron-demand Diels-Alder cycloaddition (SPIEDAC) of cyclooctynes or -octenes with tetrazines has also recently been used to site-selectively label proteins. While the latter reactions proceed with tremendous reaction rates, wide-spread application has thus far been hampered by

limited accessibility of the tetrazine probes. Another shortcoming of the vast majority of the above cycloaddition reactions is the lack of a temporally controlled activation, since these bioconjugation reactions occur spontaneously upon encountering of the reactive moieties. The only exception in this regard is the cycloaddition of alkenes and nitrile imines, which must be generated *in situ* by UV irradiation of tetrazoles, but this approach is not readily scalable and may not always be compatible with sensitive molecules due to the short-wavelength light (300 nm). Controllable, site-specific reactions are highly valuable as they enable multiplexing of (bio)molecules and also expand labeling diversity.

Hence, it is an aspect of the invention to provide an alternative method for making such conjugate, which preferably further at least partly obviate one or more of above-described drawbacks, and which especially has a high orthogonality.

Herein, it is suggested to use the potential of 1,2-quinone cycloadditions with a highly reactive cyclooctyne variant, *i.e.* bicyclo[6.1.0]non-4-yne (BCN), and similar aliphatic cycloalkynes, as a novel conjugation tool. We show that the (4+2) cycloaddition of 1,2-quinone with BCN proceeds extremely fast in aqueous media leading to the corresponding [2.2.2]-bicyclooctadiene cycloadduct. Importantly, the cycloaddition can be selectively induced by oxidation of a precursor catechol moiety to a 1,2-quinone providing temporal control over the reaction (as shown in fig. 1). Since 1,2-quinones can be readily generated in one step by oxidation of catechol, is it suggested herein to use e.g. the catechol-to-quinone conversion, and similar conversions, as a chemically activatable conjugation or connection method.

To assess the scope and limitations of the cyclooctyne-1,2-quinone reaction, first a model system was studied in detail. We selected the monosubstituted 4-tert-butyl substituted quinone (1) as a (relatively) stable and simple model substrate to investigate the reaction kinetics for cycloaddition with BCN, a readily accessible cyclooctyne recently reported by us to undergo significantly faster cycloaddition with azides than regular cyclooctynes (approximately 100 × faster). Hence, 4-*tert*-butyl-1,2-quinone (1) was prepared by oxidation of 4-*tert*-butyl-1,2-catechol with sodium periodate and isolated as a pure solid without column chromatography. Next, cycloaddition was investigated by adding 1.5 equiv. BCN (2) to a solution of 1 in methanol/water (1:1). A rapid reaction took place as indicated by a color change from red to yellow, and the formation of a new product based on analysis with thin-layer chromatography. Purification by silica column chromatography led to the isolation of product 3 as a mixture of stereoisomers with a combined isolated yield of 80%, along with a small amount of an aromatic compound, formed by a retro-Diels-Alder reaction resulting in decarbonylation of 3 (see experimental).

The apparent fast reaction of *tert*-butyl-1,2-quinone with BCN stimulated us to investigate in detail the second order rate reaction constant of this cycloaddition, which we termed strain-promoted oxidation-controlled cyclooctyne-1,2-quinone cycloaddition (SPOCQ). Initial attempts in this respect were focused on monitoring of the reaction progress with NMR, by integrating the disappearance/formation of the representative peaks. However, the reaction was too fast to monitor, *i.e.* (near) complete conversion was already observed at the first time point. Therefore, we turned our attention to UV-spectroscopy as a potential tool for measuring reaction conversion, based on the assumption that the highly conjugated 1,2-quinone system would display much enhanced absorbance with respect to the non-conjugated product 3. Indeed, UV-spectroscopy of the respective components indicated (Figure 2a) a large difference in absorbance between 1 and 3, most notably at 384 nm. We therefore monitored the (exponential) decay of the absorbance of 1 at 384 nm over time upon reaction with BCN in 1:1 methanol/water (Figure 2c). Based on these measurements, a reaction rate constant for SPOCQ was determined to be 410 ± 39 $M^{-1}s^{-1}$, in the same order of efficiency as the reaction of BCN with tetrazines (400-1200 $M^{-1}s^{-1}$) and the reaction of spirocyclopropene with nitrile imines (890 $M^{-1}s^{-1}$), but several orders of magnitude faster than strain-promoted cycloaddition of azides with a variety of cyclooctyne derivatives (typical values 0.01-1 $M^{-1}s^{-1}$). Furthermore, the cycloaddition of 1 with BCN outperforms the SPANC reaction (highest values up to 39 $M^{-1}s^{-1}$) or the recently identified reaction of BCN with sulfenic acid (12 $M^{-1}s^{-1}$). In fact, to the best of our knowledge, only the strain-promoted inverse electron-demand Diels-Alder cycloaddition of tetrazine with *trans*-cyclooctene shows a higher reaction rate constant (> 5000 $M^{-1}s^{-1}$) than SPOCQ. We also investigated the cycloaddition of **1** with cyclooctyne derivative dibenzoazacyclooctyne (DIBAC, also known as DBCO), earlier developed by us as well as by others. While DIBAC is known to react faster with (aliphatic) azides than BCN, surprisingly, the reaction rate constant for cycloaddition with 1,2-quinone was found to be slower (0.19 $M^{-1}s^{-1}$) than that for reaction with BCN, as determined by NMR, and is comparable to the rate constants for previously described SPAAC reactions (see experimental). However, also this reaction is of interest because of its inclusion in a controllable reaction (by oxidation) and its orthogonality. The latter finding is in line with the observation that 1,2,4,6-tetrazines, another type of electron-deficient diene-reagents, react quickly with BCN, but not with DIBAC.

Next, we explored the feasibility of SPOCQ for bioconjugation. First, we turned our attention to the selective labeling of peptides containing a catechol moiety. Model peptide **4,** readily generated and purified by standard (solid phase) peptide synthesis, was first oxidized for 30 min with sodium periodate (1 equiv.) to generate the corresponding 1,2-quinone *in situ,* followed by incubation with BCN-biotin (5). The progress of reaction was analyzed by high performance liquid chromatography (HPLC) using a reverse phase C-18 column. A new peak was identified with a retention time of 9 min (see experimental). Mass spectrometry analysis of the newly formed product revealed a corresponding mass matching with that of the anticipated cycloadduct product **6** (see experimental). When peptide **4** was not oxidized prior

to incubation with **5,** no reaction product was detected (see experimental). These results show that the catechol moiety is inert towards BCN and can controllably be activated by oxidation to undergo reaction with BCN.

The large difference in reaction rate constants of BCN with azide and 1,2-quinone triggered us to further investigate whether mutually orthogonal labeling could be achieved by the combination of SPOCQ and SPAAC. Thus, a competition experiment was designed with peptide **4** and N-terminally azide-functionalized peptide 7. HPLC and MS analysis of the reaction of oxidized peptide **4** with **5** and 7 revealed the formation of cycloaddition product of **4** and **5,** whereas the mass of the product from the SPAAC reaction between **5** and 7 (anticipated product **8)** was not detected (see experimental). However, when we repeated the experiment without adding the oxidized peptide, mass spectrometry confirmed the presence of the expected SPAAC product (see experimental). These findings show that the higher reaction rate of SPOCQ outcompetes SPAAC and can be used for selective labeling. Moreover, since the catechol moiety is inert towards BCN and only reacts after oxidation, it can be used as an activatable handle in conjugation studies.

Hence, it should be possible to use SPOCQ for protein labeling. Although it is known that 1,2-quinones react with naturally occurring functionalities such as amines and thiols, chemoselective labeling with SPOCQ may still be feasible, since the reaction of BCN with 1,2-quinone displays such a high reaction rate. For protein labeling purposes, BCN can be readily incorporated into proteins via genetic code expansion as has been described by us earlier. Thus, we used a C-terminally $His_6$-tagged green fluorescent protein ($GFP^{BCN}$) that carried an amber codon (TAG) mutation at position 39 as a model protein and genetically encoded BCN-lysine using a previously engineered version of the pyrrolysine tRNA-synthetase (pylRS) from *Methanosarcina mazei* and its corresponding suppressor $tRNA_{CUA}$ (Figure 4a). After recombinant expression, $GFP^{BCN}$ was purified by means of Ni-NTA chromatography. A catechol-containing fluorescent dye **(9),** synthesized in three steps from sulforhodamine B acid chloride and dihydroxyphenyl acetic acid (supp. info), was then oxidized **(10)** and subsequently incubated (4 equiv.) with $GFP^{BCN}$ at room temperature for 1 h (Figure 4b). Wild-type GFP ($GFP^{WT}$), that contains a tyrosine residue at position 39, was used as a negative control. Gratifyingly, in-gel fluorescence analysis revealed that $GFP^{BCN}$ selectively reacts with **10.** Moreover, reaction of $GFP^{BCN}$ was found to be much faster than the reaction of other nucleophiles such as amines or thiols present in proteins, which limits undesired nonspecific labeling. Next, we followed the reaction of $GFP^{BCN}$ with **10** as a function of time to estimate whether the high reaction rate of SPOCQ, observed for small molecules, also translates into fast labeling of proteins. Samples were taken at various time points and subsequently quenched with an excess of hydroxymethyl-BCN. We also performed the experiment with an azido-dye **(11)** to directly compare the labeling kinetics of 1,2-quinone cycloaddition to that of SPAAC. In-gel fluorescence analysis showed that already after 1 min, $GFP^{BCN}$ was clearly fluorescently labeled with **10** and reached saturation within a few minutes (Figure 4b). In contrast, only faint labeling of $GFP^{BCN}$ with **11** was observable and did not reach completion within 1 h. To prove that the reaction of **10** with $GFP^{BCN}$ was indeed oxidation-controlled, we incubated $GFP^{BCN}$ with **9** and did not observe any fluorescent labeling (Figure 3b).

As a first attempt towards multiplexing, we sought to use the orthogonality of SPOCQ and SPAAC to form protein dimers. We synthesized a bifunctional linker **(12,** Figure 3c containing both an azide and a catechol moiety). A small excess of the linker (5 equiv.), after oxidation with periodate, was then incubated with $GFP^{BCN}$ for 1 h at room temperature. Subsequently, the excess of linker was removed by spin filtration and another equivalent of $GFP^{BCN}$ was added, leading to the desired formation (20-30%) of protein dimer, as analyzed by gel electrophoresis and coomassie staining. As expected, no dimer formation was observed without prior oxidation of the linker. Although other studies using bifunctional linkers to form protein dimers reported slightly higher yields (up to 50%), we envisage that further optimization of dimer formation could be achieved *e.g.* by use of a longer linker or incubation at higher concentrations. Nevertheless, these experiments demonstrate the feasibility of selective activation-conjugation by a combination of SPOCQ and SPAAC for multiplexing. Finally, it must be noted that the formed dimers were readily separated from the unreacted monomers with size exclusion chromatography, yielding a population of well-defined protein dimers (see experimental).

Besides the use in bioconjugation, we also explored the possibilities to use the SPOCQ reaction for the synthesis of hydrogels. We modified 4-arm star poly(ethylene glycol) with either BCN or catechol moieties. After oxidation of the catechol to the quinone using periodate, a very fast gelation reaction was observed, even at polymer concentrations as low as 1%. The oxidation was also performed with mushroom tyrosinase. Again, at low enzyme dosages of 250 U, crosslinking was completed with 5 minutes reaction time. This demonstrates the versatility of the SPOCQ reaction which can be equally efficiently used in bioconjugation as materials science.

In conclusion, we have described a new (bio)conjugation reaction based on cycloaddition of bicyclo[6.1.0]nonyne and 1,2 benzoquinone derivatives. This new cycloaddition, which we coin SPOCQ (strain-promoted oxidation-controlled cyclooctyne 1,2-quinone cycloaddition) shows an extremely high reaction rate constant, several orders of magnitude faster than the SPAAC. Hence, the 1,2-quinone (or other electron-poor dienes) and azide can be used as an orthogonal reaction pair, which may be useful for multiplexing applications in protein labeling. Furthermore, the option of selective induction of SPOCQ by the oxidation of catechol (and other precursors of electron-poor dienes) to the corresponding 1,2-quinone (or other electron-poor dienes, respectively) adds a new temporally controlled and fast reaction to the (bio)conjugation toolbox, which is particularly likely in the light that both BCN as well as catechol-bearing amino acids can be genetically incorporated into proteins. In addition it may be postulated that selective detection of oxidative cellular environments (hypoxia) may become feasible by using appropriate catechol-bearing reporter molecules (e.g. fluorophores). We generated all 1,2-quinone derivatives using sodium periodate. However,, the oxidation can also be mediated by other oxidizing agents as well as enzymes (e.g. catechol oxidase), providing more selectivity for the oxidation step. Finally, it is expected that the fact that a variety of catechol derivatives as well as BCN-derivatives are readily accessible, diverse applications of SPOCQ will be further facilitated, in the context of bioconjugation and beyond.

Hence, in a first aspect the use of a (hetero)cycloalkyne and an oxo-group containing (hetero)1,3-diene for coupling a first system (A) of interest to a second system (A') of interest, wherein a (hetero)cycloalkyne is created in one of the first system (A) of interest and the second system (A') of interest, and wherein an oxo-group containing (hetero)1,3-diene is created in the other system of interest, and allowing the (hetero)cycloalkyne and the oxo-group containing (hetero)1,3-diene to react to form an A-A' conjugate. Hereby, an orthogonal reaction pair is provided, that can be used with high selectivity to couple systems of interest to a conjugate.

In a further aspect, the invention provides a method (herein also indicated as "process") for the oxidation-induced production of a conjugate defined by formula III from a first reaction partner comprising a (hetero)cycloalkyne defined by formula I and a second reaction partner comprising an oxo-group containing (hetero)1,3-diene defined by formula II according to the following scheme:

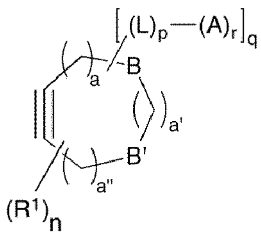

(I)

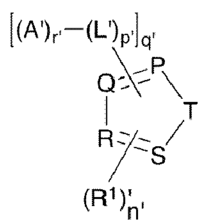

(II)

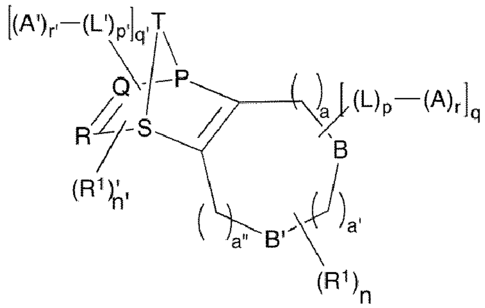

(III)

wherein the method especially comprises (i) combining the first reaction partner and the second reaction partner comprising an oxidizable precursor of the (oxo-group containing) (hetero)1,3-diene, (ii) oxidizing before combining, during combining or after combining, especially after combining, the oxidizable precursor of the (oxo-group containing) (hetero)1,3-diene to the oxo-group containing (hetero)1,3-diene defined by formula II, and (iii) allowing the (hetero)cycloalkyne and the oxo-group containing (hetero)1,3-diene to react to form the conjugate defined by formula III, wherein:

a, a' and a" are independently selected from 0 - 8, with the proviso that a + a' + a" = 4, 5, 6, 7 or 8;
n is 0-16, especially 0-4; n' is 0-16, especially 0-4;
$R^1$ and $R^{1'}$ are independently selected from the group consisting of oxo, halogen, $-OR^2$, $-NO_2$, -CN, $-S(O)_2R^2$, $C_1$ - $C_{24}$ alkyl groups, $C_3$ - $C_{24}$ cycloalkyl groups, $C_2$ - $C_{24}$ (hetero)aryl groups, $C_3$ - $C_{24}$ alkyl(hetero)aryl groups and $C_3$ - $C_{24}$ (hetero)arylalkyl groups, wherein the alkyl groups, cycloalkyl groups, (hetero)aryl groups, alkyl(hetero)aryl groups and (hetero)arylalkyl groups are optionally substituted, wherein the alkyl groups, cycloalkyl groups, alkyl(hetero)aryl groups and (hetero)arylalkyl groups are optionally interrupted by one or more heteroatoms selected from the group consisting of O, S and N, and wherein $R^2$ is independently selected from the group consisting of hydrogen, halogen, $C_1$ - $C_{24}$ alkyl groups, $C_3$ - $C_{24}$ cycloalkyl groups, $C_2$ - $C_{24}$ (hetero)aryl groups, $C_3$ - $C_{24}$ alkyl(hetero)aryl groups and $C_3$ - $C_{24}$ (hetero)arylalkyl groups;
B and B' are independently selected from the group consisting of O, S, C(O), $NR^3$ and $C(R^3)_2$, wherein $R^3$ is independently selected from the group consisting of hydrogen, $R^1$ or $(L)_p$-$(A)_r$;

optionally, when n is 2 or more, two $R^1$ groups may together form a (hetero)cycloalkyl group, the (hetero)cycloalkyl group optionally being substituted with an $(L)_p$-$(A)_r$ substituent;
optionally, when n is 2 or more, two $R^1$ groups may together form a (hetero)aryl group, the (hetero)aryl group optionally being substituted with an $(L)_p$-$(A)_r$ substituent;
optionally, when n' is 2 or more, two $R^{1'}$ groups may together form a (hetero)cycloalkyl group, the (hetero)cycloalkyl group optionally being substituted with an $(L)'_p$-$(A)'_r$ substituent;

p is 0 or 1; p' is 0 or 1;
r is 1 - 4; r' is 1 - 4;
L is a linker; L' is a linker;
A is a first system of interest to be connected to a second system A' of interest;

q is 0 - 4, especially 1 - 4; q' is 0 - 4, especially 1 - 4;

with the proviso that if q is 0, then B and/or B' is $NR^3$ wherein $R^3$ is $(L)_p$-$(A)_r$, and/or B and/or B' is $C(R^3)_2$ wherein one or more $R^3$ is $(L)_p$-$(A)_r$, and/or n is 2 or more and two $R^1$ groups together form a (hetero)cycloalkyl group wherein the (hetero)cycloalkyl group is substituted with an $(L)_p$-$(A)_r$ substituent, and/or n is 2 or more and two $R^1$ groups together form a (hetero)aryl group wherein the (hetero)aryl group is substituted with an $(L)_p$-$(A)_r$ substituent;

P,Q,R,S are independently selected from C or N; and
T comprises an oxo-group selected from the group consisting of:

-C(O)C(O)-;

-C(O)C(=NR*), with R* being selected from the group consisting of H and alkyl and aryl;

-C(O)-;

-OC(O)-;

-S(O)-;

and

$-SO_2-$.

The current invention entails an activatable reaction which meets all the requirements of a click reaction. The reaction uses as reaction partners bicyclononyne (BCN), a bicyclic structure based on cyclooctyne, and 1,2-quinone, or in other words the oxidized, diketo form of 1,2-catechol and related molecules. Oxidation can be performed chemically, by using periodate, or enzymatically using naturally occurring tyrosinase or catalase. Oxidation is achieved with full conversion. Only after oxidation does reaction occur. The reaction between the partners is the second fastest click reaction known after reaction (>500 M-1s-1) known after SPIEDAC (>2000 M-1s-1), and is highly orthogonal; only slow interference with other functionalities present in biomolecules are found. The reaction outcompetes the SPAAC reaction between bicyclononyne and azide by more than a factor 1000. If both the diketo compound and azide are present in equimolar amounts, full conversion with the diketo compound is achieved, leaving the azide untouched.

The 1,2-catechol unit is easily introduced in peptides or proteins in the form of 3,4-dihydroxyphenylalanine (DOPA), either by chemical synthesis (peptides) or by posttranslational modification or by protein engineering (proteins). Since the starting amino acid compound and 1,2-catechols in general are readily available, the moiety can be incorporated in any desired organic structure stretching beyond peptides and proteins, e.g. sugars or nucleic acids, but also any functional labeling agent, such as biotin or fluorophores. There is much interest in using polydopamine films as adhesive layers for for example cell culture experiments.

The invention thus describes a conceptually novel approach to what is known in literature. No other oxidation-induced strain-promoted cycloadditions are known for the purpose of conjugation and the efficiency and rate of the reaction are unexpected. This is absolutely necessary to make this reaction into a success. As a comparison, similar strained alkynes, such as plain cyclooctyne, react much slower with 1,2-quinone. In addition, we established ourselves that a benzofused cyclooctyne as for example dibenzoazacyclooctyne (DIBAC/DBCO) reacts slower than BCN. This shows that it is unpredictable that BCN reacts so well and selective with 1,2-quinone compounds.

Further, the controllability of the process due to the oxidation, which can e.g. be performed in situ, is a great advantage over state of the art processess, which may start upon combining the reaction partners. With the present invention it is possible to combine the reaction partners, with one being a precursor of the real reaction partner, executing the oxidation, and letting the reaction partners to react.

Prior art solutions require more elaborate chemistry to obtain the requisite reaction partners which hampers ease of application. The coupling method described is quite robust and could thus be well standardized. A reaction between 1,2-quinone with cyclooctyne with such high reaction rate constant is hitherto unknown.

As indicated above, compared to click chemistry approaches, the addressability of one of the reaction partners is of high advantage. Only upon oxidation of the dihydroxyphenyl moiety to the 1,2-quinone, reaction occurs, which allows premixing of both reaction components that will only react with each other upon an oxidative trigger. This reaction is furthermore the second fastest click reaction reported. This makes this reaction better suitable for in vitro and in vivo applications at high dilution than most of the other click chemistry approaches. Compared to dopamine chemistry this process is fully controlled with regard to reaction partners and product obtained. As a result a click chemistry reaction is obtained which allows for spatiotemporal control over the occurrence of the reaction. Other advantages are the easy access to the reaction partners and the fact that one of the two reactive groups can be found/introduced efficiently in biological systems.

Hence, the invention also provides the product obtainable by the herein described method. Therefore, in a further aspect the invention also provides a conjugate defined by formula III:

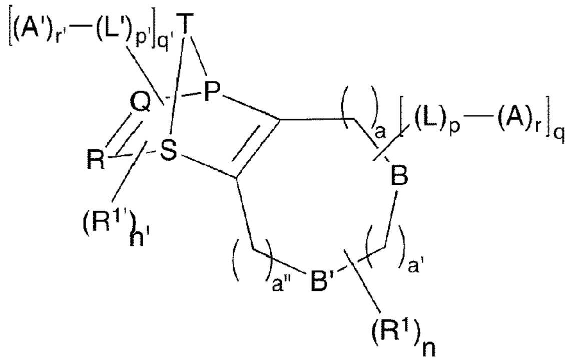

(III)

wherein A, A', L, L', p,p', $R^1$, $R^{1'}$, r, r', q,q',B,B', a, a', a", P, Q, R, S, T are as defined herein.

BRIEF DESCRIPTION OF THE DRAWINGS

Embodiments of the invention will now be described, by way of example only, with reference to the accompanying schematic drawings in which corresponding reference symbols indicate corresponding parts, and in which:

Figure 1 shows a first scheme: strain-promoted alkyne-azide cylcoaddition with BCN and strain-promoted oxidation-controlled cyclooctyne-1,2-quinone cycloaddition. Spheres indicate the systems of interst A or A', respectively;

Figure 2 shows the reaction kinetics of SPOCQ. a). Cycloaddition of 4-*tert*-butyl-1,2-quinone (1) with BCN (2). b) UV/Vis spectrum of 1 and SPOCQ product 3 and determination of reaction rate constant in MeOH/$H_2O$ (1:1) at room temperature using decay in absorbance of 1 at 384 nm;

Figure 3 shows a competition experiment of catechol- and azido-functionalized peptide (4 and 7, respectively) with BCN-biotin (5). Competition experiment with and without oxidized peptide 4 was analyzed by HPLC followed by ESI-MS;

Figure 4 shows SPOCQ for bioconjugation. a) Site-specific incorporation of $GFP^{BCN}$ into GFP. b) In-gel fluorescence assay and corresponding coomassie stained protein gels of fluorescently labelled $GFP^{BCN}$ using SPOCQ and SPAAC. c) Protein gel showing controlled dimer formation of $GFP^{BCN}$ using bifunctional linker 2 after oxidation. Dimers were separated from monomers using size exclusion chromatography.

Figure 5 schematically depicts a (hydro)gel, with (former) reaction partners A and A' coupled via the group III. Of course, other linkages and combinations of reaction partners are also possible. This is a very schematic drawing.

DETAILED DESCRIPTION OF THE EMBODIMENTS

As indicated above, the invention provides a method for the oxidation-induced production of a conjugate defined by formula III from a first reaction partner comprising a (hetero)cycloalkyne defined by formula I and a second reaction partner comprising an oxo-group containing (hetero)1,3-diene defined by formula II according to the following scheme:

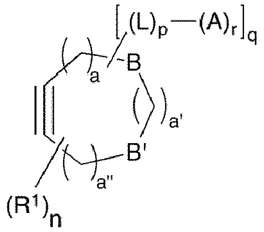
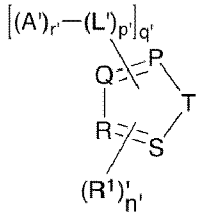
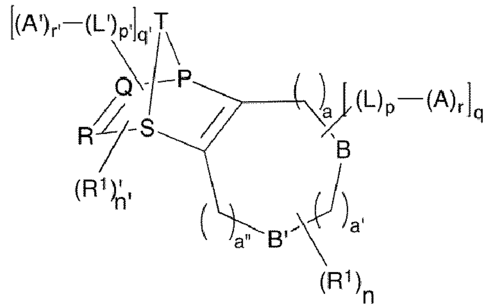

(I) (II) (III)

wherein the method comprises (i) combining the first reaction partner and the second reaction partner comprising an oxidizable precursor of the (oxo-group containing) (hetero)1,3-diene, (ii) oxidizing before combining, during combining or after combining, especially after combining, the oxidizable precursor of the (oxo-group containing) (hetero)1,3-diene to the oxo-group containing (hetero)1,3-diene defined by formula II, and (iii) allowing the (hetero)cycloalkyne and the oxo-group containing (hetero)1,3-diene to react to form the conjugate defined by formula III, wherein:

a, a' and a" are independently selected from 0 - 8, with the proviso that a + a' + a" = 4, 5, 6, 7 or 8;
n is 0-16, especially 0-4; n' is 0-16, especially 0-4;
$R^1$ and $R^{1'}$ are independently selected from the group consisting of oxo, halogen, $-OR^2$, $-NO_2$, -CN, $-S(O)_2R^2$, $C_1$ - $C_{24}$ alkyl groups, $C_3$ - $C_{24}$ cycloalkyl groups, $C_2$ - $C_{24}$ (hetero)aryl groups, $C_3$ - $C_{24}$ alkyl(hetero)aryl groups and $C_3$ - $C_{24}$ (hetero)arylalkyl groups, wherein the alkyl groups, cycloalkyl groups, (hetero)aryl groups, alkyl(hetero)aryl groups and (hetero)arylalkyl groups are optionally substituted, wherein the alkyl groups, cycloalkyl groups, alkyl(hetero)aryl groups and (hetero)arylalkyl groups are optionally interrupted by one or more heteroatoms selected from the group consisting of O, S and N, and wherein $R^2$ is independently selected from the group consisting of hydrogen, halogen, $C_1$ - $C_{24}$ alkyl groups, $C_3$ - $C_{24}$ cycloalkyl groups, $C_2$ - $C_{24}$ (hetero)aryl groups, $C_3$ - $C_{24}$ alkyl(hetero)aryl groups and $C_3$ - $C_{24}$ (hetero)arylalkyl groups;
B and B' are independently selected from the group consisting of O, S, C(O), $NR^3$ and $C(R^3)_2$, wherein $R^3$ is independently selected from the group consisting of hydrogen, $R^1$ or $(L)_p$-$(A)_r$;

optionally, when n is 2 or more, two $R^1$ groups may together form a (hetero)cycloalkyl group, the (hetero)cycloalkyl group optionally being substituted with an $(L)_p$-$(A)_r$ substituent;
optionally, when n is 2 or more, two $R^1$ groups may together form a (hetero)aryl group, the (hetero)aryl group optionally being substituted with an $(L)_p$-$(A)_r$ substituent;
optionally, when n' is 2 or more, two $R^{1'}$ groups may together form a (hetero)cycloalkyl group, the (hetero)cycloalkyl group optionally being substituted with an $(L)'_p$-$(A)'_r$ substituent;

p is 0 or 1; p' is 0 or 1;
r is 1 - 4; r' is 1 - 4;
L is a linker; L' is a linker;
A is a first system of interest to be connected to a second system A' of interest;

$q \geq 0$; especially q is 1 - 4; $q' \geq 0$; especially q' is 1 - 4;
with the proviso that if q is 0, then B and/or B' is $NR^3$ wherein $R^3$ is $(L)_p$-$(A)_r$, and/or B and/or B' is $C(R^3)_2$ wherein one or more $R^3$ is $(L)_p$-$(A)_r$, and/or n is 2 or more and two $R^1$ groups together form a (hetero)cycloalkyl group wherein the (hetero)cycloalkyl group is substituted with an $(L)_p$-$(A)_r$ substituent, and/or n is 2 or more and two $R^1$ groups together form a (hetero)aryl group wherein the (hetero)aryl group is substituted with an $(L)_p$-$(A)_r$ substituent;

P,Q,R,S are independently selected from C or N; and
T comprises an oxo-group selected from the group consisting of:

-C(O)C(O)-;

-C(O)C(=NR*), with R* being selected from the group consisting of H and alkyl and aryl group;

-C(O)-;

-OC(O)-;

-S(O)-;

and

$-SO_2-$.

As indicated above, A is a first system of interest to be connected to a second system A' of interest. These systems can be any systems that one desires to couple. In general, at least one of the systems comprises a (bio) organic molecule, such as a protein, a peptide, an enzyme, etc. Further, especially the two systems may especially include a host material and a functional molecule, such as a reporter molecule, a dye, a marker, etc.. Further, the first system of interest and the second system of interest may be used to make (hydro)gels.

To discriminate between different options, the first systems A and second system A' are below describe in more detail with respect to a number of options. In an embodiment, A and A' are independently selected from the group consisting of D, E and G, wherein D comprises a molecule of interest, E comprises a polymer or solid surface, and G comprises a functional group. In general, $A \neq A'$. The terms "first" and "second" in this context are only used to distinguish the systems. Further, when one of A and A' includes E, especially the other of A and A' includes D or G. Note that more than two systems may be coupled. Also a plurality of (different) systems may be coupled. Here, for the sake of understanding the product and reaction are only described with reference to the first system and the second system, though more systems (and this links) are also part of the invention. Such systems may be coupled with the same SPOCQ type of process or with another process.

For instance, in an embodiment, D is independently selected from the group consisting of a reporter molecule, an active substance, an enzyme, a protein, a glycoprotein, an antibody, a peptide, a polypeptide, an oligonucleotide, a monosaccharide, an oligosaccharide, a polysaccharide, a glycan, a diagnostic compound, an amino acid, a (poly)ethylene glycol diamine, a polyethylene glycol chain, a polyethylene oxide chain, a polypropylene glycol chain, a polypropylene oxide chain, a polyoxazoline chain, poly oligoethylene glycol(meth)acrylate chain, a poly(N-alkyl acrylamide) chain, and a 1,x-diaminoalkane (wherein x is the number of carbon atoms in the alkane).

A reporter molecule may especially be a molecule whose presence is readily detected, for example a diagnostic agent, a dye, a fluorophore, a radioactive isotope label, a contrast agent, a magnetic resonance imaging agent or a mass label. Examples of a fluorophore include all kinds of Alexa Fluor (e.g. Alexa Fluor 555), cyanine dyes (e.g. Cy3 or Cy5), coumarin derivatives, fluorescein, rhodamine, allophycocyanin and chromomycin.

An active substance may especially be a pharmacological and/or biological substance, i.e. a substance that is biologically and/or pharmaceutically active, for example a drug or a prodrug, a diagnostic agent, an amino acid, a protein, a peptide, a polypeptide, a monosaccharide, an oligosaccharide, a polysaccharide, a glycan, a lipid, a vitamin, a steroid, a nucleotide, a nucleoside, a polynucleotide, RNA or DNA. Examples of suitable peptide tags include a cell-penetrating peptides like human lactoferrin or polyarginine. An example of a suitable glycan is oligomannose. Preferably, the active substance is selected from the group consisting of drugs and prodrugs. More preferably, the active substance is selected from the group consisting of pharmaceutically active compounds, in particular low to medium molecular weight compounds (e.g. about 200 to about 1500 Da, preferably about 300 to about 1000 Da), such as for example cytotoxins, antiviral agents, antibacterials agents, peptides and oligonucleotides. Examples of cytotoxins include colchicine, vinca alkaloids, camptothecins, doxorubicin, daunorubicin, taxanes, calicheamycins, duocarmycins, maytansines, auristatins, tubulysin, irinotecans, an inhibitory peptide, amanitin, deBouganin, or pyrrolobenzodiazepines (PBDs).

Examples of radioactive isotope label include $^{99m}Tc$, $^{111}In$, $^{18}F$, $^{14}C$, $^{64}Cu$, $^{131}I$ or $^{123}I$, which may or may not be connected via a chelating moiety such as DTPA, DOTA, NOTA or HYNIC.

A solid surface E may especially be a functional surface (e.g. nanomaterials, carbon nanotubes, fullerenes, virus capsids), metal surface (e.g. gold, silver, copper, nickel, tin, rhodium, zinc) or a metal alloy surface (from aluminium, bismuth, chromium, cobalt, copper, gallium, gold, indium, iron, lead, magnesium, mercury, nickel, potassium, plutonium, rhodium, scandium, silver, sodium, titanium, tin, uranium, zinc, zirconium), a synthetic polymer surface (e.g. polystyrene, polyvinylchloride, polyethylene, polypropylene, poly(dimethylsiloxane), polymethylmethacrylate, polyamide, polyester, polyacrylonitrile) and biopolymer surface (e.g. cellulose, chitin, wool), an inorganic surface (glass, quartz, silicon, silicon nitride). E is preferably independently selected from the group consisting of a functional surface or a polymer surface.

A water-soluble polymer E may especially be a synthetic polymer capable of multifunctionalization in order to form a hyperbranched structure, a network, a hydrogel, an interpenetrating network (e.g. poly (ethylene glycol or poly(ethylene oxide), poly (oligoethylene glycol(meth)acrylate), poly(N-alkyl acrylamide), poly(meth)acrylic acid, poly (ethylene imine), polyamides, polyesters, polycarbonates, polyanhydrides), or polymer E is a natural polymer capable of multifunctionalization, (e.g. cellulose, chitosan, hyularonic acid, amylose, dextran, collagen, elastin or elastin-like polypeptides, globular proteins), or E is a low molecular weight analogue of the abovemnentioned polymers, (e.g. oligoethylene glycol, alkanediamine, oligopeptide) or E is a combination of the abovementioned structures.

A functional group G may especially independently be selected from the group consisting of hydrogen, halogen, $R^{11}$, $-CH=C(R^{11})_2$, $-C{\equiv}CR^{11}$, $-[C(R^{11})_2C(R^{11})_2O]_q-R^{11}$, wherein q is in the range of 1 to 200, CN, $N_3$, -BCX, XCN, $-XR^{11}$, $-N(R^{11})_2$, $-^{+}N(R^{11})_3$, $-C(X)N(R^{11})_2$, $-C(R^{11})_2XR^{11}$, $-C(X)R^{11}$, $-C(X)XR^{11}$, $-S(O)R^{11}$, $-S(O)_2R^{11}$, $-S(O)OR^{11}$, $-S(O)_2OR^{11}$, $-S(O)N(R^{11})_2$, $-S(O)_2N(R^{11})_2$, $-OS(O)R^{11}$, $-OS(O)_2R^{11}$, $-OS(O)OR^{11}$, $-OS(O)_2OR^{11}$, $-P(O)(R^{11})(OR^{11})$, $-P(O)(OR^{11})_2$, $-OP(O)(OR^{11})_2$, $-Si(R^{11})_3$, $-XC(X)R^{11}$, $-XC(X)XR^{11}$, $-XC(X)N(R^{11})_2$, $-N(R^{11})C(X)R^{11}$, $-N(R^{11})C(X)XR^{11}$ and $-N(R^{11})C(X)N(R^{11})_2$, wherein X is oxygen or sulphur and wherein $R^{11}$ is independently selected from the group consisting of hydrogen, halogen, $C_1$ - $C_{24}$ alkyl groups, $C_3$ - $C_{24}$ cycloalkyl groups, $C_2$ - $C_{24}$ (hetero)aryl groups, $C_3$ - $C_{24}$ alkyl(hetero)aryl groups and $C_3$ - $C_{24}$ (hetero)arylalkyl groups, the $C_1$ - $C_{24}$ alkyl groups, $C_3$ - $C_{24}$ cycloalkyl groups, $C_2$ - $C_{24}$ (hetero)aryl groups, $C_3$ - $C_{24}$ alkyl(hetero)aryl groups and $C_3$ - $C_{24}$ (hetero)arylalkyl groups optionally substituted and optionally interrupted by one or more heteroatoms selected from O and N.

Especially, $R^{11}$ (of the G-group(s)) is independently selected from the group consisting of hydrogen, halogen and $C_1$ - $C_6$ alkyl groups, more preferably from the group consisting of hydrogen, halogen and $C_1$ - $C_4$ alkyl groups. Most preferably, $R^1$ is independently selected from the group consisting of hydrogen, methyl, ethyl, propyl, i-propyl, butyl and *t*-butyl. X is preferably oxygen.

Optionally, functional group G is masked or protected. More preferably, G is independently selected from the group consisting of -CN,-NCX, -XCN, $-XR^{11}$, $-N(R^{11})_2$, $-^{+}N(R^{11})_3$, $-C(X)N(R^{11})_2$, $-C(R^{11})_2XR^{11}$, $-C(X)R^{11}$, $-C(X)X R^{11}$, $-XC(X)R^{11}$, $-XC(X)XR^{11}$, $-XC(X)N(R^{11})_2$, $-N(R^{11})C(X)R^{11}$, $-N(R^{11})C(X)XR^{11}$ and $-N(R^{11})C(X)N(R^{11})_2$, wherein X and R", and preferred embodiments of X and R", are as defined above. Most preferably, G is selected from the group consisting of - OR", -SR", $-N(R^{11})_2$, $-^{+}(R^{11})_3$, $-C(O)N(R^{11})_2$, $-C(O)OR^{11}$, $-OC(O)R^{11}$, $-OC(O)OR^{11}$, $-O C(O)N(R^{11})_2$, $-N(R^{11})C(O)R^{11}$, $-N(R^{11})C(O)OR^{11}$ and $-N(R^{11})C(O)N(R^{11})_2$, wherein X and R", and preferred embodiments of X and R", are as defined above. As is known in the art, C(O) indicates a carbon with a double bond oxygen, i.e. C=O.

In a specific embodiment, the invention provides e.g. one or more of a a protein-label conjugate, a protein-protein conjugate, a proteint-peptide conjugate, a protein- polymer conjugate, a protein-nanoparticle conjugate, a polymer network, a polymer hydrogel, and an antibody-drug conjugate. For instance, the invention may provide a protein with a label, a protein-protein conjugate, a protein with a carbohydrate moiety, a protein with a bioactive peptide (such as a cell-penetrating peptide), a protein with a polymer conjugate (such as poly(ethylene glycol), poly(oxazolines), poly(N-isopropyl acrylamide)), a protein with a nanoparticle (such as a liposme, a polymer micelle, a vesicle, a metal nanoparticles), a block copolymer conjugate (di,tri and multiblock copolymer, a polymer-peptide conjugate (both a main chain conjugate or as side-chain conjugate), a polymer network or a hybrid polymer network (like a polymer hydrogel, an interpenetrating network, a polymer network functionalized with a protein and/or a peptide, etc. Further, the invention can be used for e.g. protein, peptide and polymer immobilization (on a surface, such as applied in biosensors (ELISA, SPR) and microarrays, in bulk material, for example in hydrogels which can be used for biomedical applications such as tissue engineering and controlled drug delivery, and on polymer carriers to e.g. protect, concentrate and assemble enzymes, on functional surfaces (e.g. nanomaterials, carbon nanotubes, fullerenes, virus capsids), metal surface (e.g. gold, silver, copper, nickel, tin, rhodium, zinc) or a metal alloy surface (from aluminium, bismuth, chromium, cobalt, copper, gallium, gold, indium, iron, lead, magnesium, mercury, nickel, potassium, plutonium, rhodium, scandium, silver, sodium, titanium, tin, uranium, zinc, zirconium), a synthetic polymer surface (e.g. polystyrene, polyvinylchloride, polyethylene, polypropylene, poly(dimethylsiloxane), polymethylmethacrylate, polyamide, polyester, polyacrylonitrile) and biopolymer surface (e.g. cellulose, chitin, wool), an inorganic surface (glass, quartz, silicon, silicon nitride). E is preferably independently selected from the group consisting of a functional surface or a polymer surface).

Hence, amongst others, the invention may be applied in two-dimensional gel electrophoresis separation, preparative organic chemistry (click chemistry), modification of peptides, modification of natural products, modification of pharmaceuticals, drug discovery, modification of DNA or nucleotides, supramolecular chemistry, dendrimer design, (bio)polymer (modification), surface modification, material modification, nanotechnology, bioconjugation, bioorthogonal labeling and 3D imaging of living cells, synthesis and gelation of (DOPA-modified) PEG hydrogels, etc. Also in vivo reactions are possible with the novel method, due to its high reaction rate, high orthogonality, and switchability (starting the reaction by oxidation). Further, the method of the invention may especially of interest for a (poly)dopamine containing reaction partner or (poly)dopamine functionalized reaction partner, because of the 1,2-diphenol group.

Hence, as amongst others defined in WO2014/065661 a molecule of interest may for example be a reporter molecule, an active substance, an enzyme, a (non-catalytic) protein, a peptide, an oligonucleotide, a glycan, an azide or a (hetero)cycloalkynyl moiety, preferably a bivalent or bifunctional (hetero)cycloalkynyl moiety. Further examples of a molecule of interest include a diagnostic compound, an amino acid (including an unnatural amino acid), a polypeptide, a (poly)ethylene glycol diamine (e.g. 1,8-diamino-3,6-dioxaoctane or equivalents comprising longer ethylene glycol chains), a polyethylene glycol chain, a polyethylene oxide chain, a polypropylene glycol chain, a polypropylene oxide

chain and l,x-diaminoalkane (wherein x is the number of carbon atoms in the alkane). In a preferred embodiment, the molecule of interest is selected from the group consisting of an amino acid (in particular lysine), an active substance, a reporter molecule, an azide and a (hetero)cycloalkynyl moiety. In a further preferred embodiment, the molecule of interest is selected from the group consisting of an active substance, a reporter molecule, an azide and a (hetero)cycloalkynyl moiety. An active substance is a pharmacological and/or biological substance, i.e. a substance that is biologically and/or pharmaceutically active, for example a drug or a prodrug, a diagnostic agent, a protein, a peptide, an amino acid, a glycan, a lipid, a vitamin, a steroid, a nucleotide, a nucleoside, a polynucleotide, RNA or DNA. Examples of suitable peptide tags include a cell-penetrating peptides like human lactoferrin or polyarginine. An example of a suitable glycan is oligomannose. In a preferred embodiment, the active substance is selected from the group consisting of drugs and prodrugs. More preferably, the active substance is selected from the group consisting of pharmaceutically active compounds, in particular low to medium molecular weight compounds (e.g. about 200 to about 1500 Da, preferably about 300 to about 1000 Da), such as for example cytotoxins, antiviral agents, antibacterials agents, peptides and oligonucleotides. Examples of cytotoxins include camptothecins, staurosporin, doxorubicin, daunorubicin, colchicine, methotrexate, taxanes, calicheamycins, duocarmycins, maytansines and maytansinoids (i.e. maytansine derivatives), auristatins, tubulysin M, cryptophycin or pyrrolobenzodiazepines (PBDs). Examples of auristatins include dolastatin 10, auristatin F, monomethyl auristatin F (MMAF), auristatin E, monomethyl auristatin E (MMAE), auristatin PE, auristatin TP and auristatin AQ. Examples of maytansines and maytansinoids include mertansine and ansamitocin.

The first and second system of interest can be coupled to each other via the diene and the cycloalkyne. These groups can be incorporated in the systems of interest and/or can be provided to the systems of interest. This can be done with methods known in the art.

In general, between the first system and the reactive group (or where relevant its precursor), and between the second system, and the other reactive group, there may be a linker, i.e. q and q' are equal to or larger than 1, respectively, and p and p' are equal to and larger than 1, respectively. When the reactive group (including in the case of the diene its precursor) is associated to one system of interest, r or r' are 1. In general, r and r' are 1. The reactive group (or where relevant its precursor) may be associated to the system of interest via more than one linkers. Hence, p and p'may be larger than 1. In general, however, p and p' are both independently 1 or 2, especially 1. When q and/or q' are larger than one, the reactive group (or where relevevant its precursor) may be bound via different linkers to the same system of interest, but the reactive group (or where relevevant its precursor) may also be bound to a plurality of different systems of interest. In general, when there is more than one system of interest coupled to the reactive group (or where relevant its precursor), each system of interest and the reactive group (or where relevant its precursor) are associated via a specific linker. Especially, p=r=q and/or p'=r'=q'.

The linker L or L' may for example be selected from the group consisting of linear or branched $C_1$-$C_{200}$ alkylene groups, $C_2$-$C_{200}$ alkenylene groups, $C_2$-$C_{200}$ alkynylene groups, $C_3$-$C_{200}$ cycloalkylene groups, $C_5$-$C_{200}$ cycloalkenylene groups, $C_8$-$C_{200}$ cycloalkynylene groups, $C_7$-$C_{200}$ alkylarylene groups, $C_7$-$C_{200}$ arylalkylene groups, $C_8$-$C_{200}$ arylalkenylene groups, $C_9$-$C_{200}$ arylalkynylene groups. Optionally the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups may be substituted, and optionally said groups may be interrupted by one or more heteroatoms, preferably 1 to 100 heteroatoms, said heteroatoms preferably being selected from the group consisting of O, S and $NR^5$, wherein $R^5$ is independently selected from the group consisting of hydrogen, halogen, $C_1$ - $C_{24}$ alkyl groups, $C_6$ - $C_{24}$ (hetero)aryl groups, $C_7$ - $C_{24}$ alkyl(hetero)aryl groups and $C_7$ - $C_{24}$ (hetero)arylalkyl groups. Most preferably, the heteroatom is O. Hence, in a specific embodiment (linkers) L and L' are independently selected from the group consisting of $C_1$-$C_{200}$ alkylene groups, $C_2$-$C_{200}$ alkenylene groups, $C_2$-$C_{200}$ alkynylene groups, $C_3$-$C_{200}$ cycloalkylene groups, $C_5$-$C_{200}$ cycloalkenylene groups, $C_8$-$C_{200}$ cycloalkynylene groups, $C_7$-$C_{200}$ alkylarylene groups, $C_7$-$C_{200}$ arylalkylene groups, $C_8$-$C_{200}$ arylalkenylene groups, and $C_9$-$C_{200}$ arylalkynylene groups, which may be substituted, unsubstituted, interrupted by one or more heteroatoms, branched or unbranched

Examples of suitable linking units include (poly)ethylene glycol diamines (e.g. 1,8-diamino-3,6-dioxaoctane or equivalents comprising longer ethylene glycol chains), polyethylene glycol or polyethylene oxide chains, polypropylene glycol or polypropylene oxide chains and l,x-diaminoalkanes wherein x is the number of carbon atoms in the alkane. Another class of suitable linkers comprises cleavable linkers. Cleavable linkers are well known in the art, which comprise self- immolative moieties that are released upon a biological trigger, e.g. an enzymatic cleavage or an oxidation event. Some examples of suitable cleavable linkers are peptide-linkers that are cleaved upon specific recognition by a protease, e.g. cathepsin, plasmin or metalloproteases, or glycoside-based linkers that are cleaved upon specific recognition by a glycosidase, e.g. glucoronidase, or nitroaromatics that are reduced in oxygen-poor, hypoxic areas. Suitable linkers are e.g. described in US2013/0137763 and WO2014/065661, which are herein incorporated by reference.

The reactive groups comprise a cycloalkyne an an electron-poor diene (which can also be indicated as a diene harboring electron-withdrawing substituents). Both can be heteromolecules or hetero groups. Hence, these groups are herein also indicated as (hetero)cycloalkyne and an electron-poor (hetero)diene. The (hetero)diene is especially an (hetero)1,3-diene. In a specific embodiment, the diene comprises an oxo-group containing (hetero)1,3-diene. The term

"oxo group" (or similar terms) refers to a double bonded O-group ("=O"), associated with a carbon or nitrogen or other atom (such as e.g. S). Especially, the oxo-group is bonded to a carbon atom: C=O. Alternatively or additionally, the (hetero)1,3-diene comprises an imino group. The term "imino group" (or similar terms) refers to a double bonded N-group ("=NR"; with R being H or alkyl or aryl group), associated with a carbon Herein, the invention is especially explained in relation to the oxo-group containing (hetero)1,3-diene (above defined awith formula II).

The oxo-group containing (hetero)1,3-diene (or other electron-poor diene) has the advantage that it can be provided by an oxidation reaction. Hence, for instance a substantially unreactive, or at least substantially non-reactive with the cyclooctyne (see also below) electron-poor diene can be provided, which can then, at a desired moment, be "switched on" to provide a reactive electron-poor diene. For instance, an 1,2-catechol group containing molecule or system of interest may be provided, that can be combined with the cyclooctyne, without substantial reaction. Upon oxidation of the 1,2-catechol containing molecule or system of interest into a quinone, the reactive groups can react. The electron-poor diene (II) and the cyclooctyne (I) are the reactive groups, which can e.g. react according to the above indicated scheme into the conjugage with formula III (see further also below).

The relevant group at the electron-poor diene is especially the T-group which comprises an oxo-group selected from the group consisting of:

-C(O)C(O)-;

-C(O)C(=NR*), with R* being selected from the group consisting of H and alkyl and aryl group;

-C(O)-;

-OC(O)-;

-S(O)-;

and

$-SO_2-$.

Each of these groups comprise an oxo or =O group, with the first mentioned group comprising two of such oxo-groups. Hence, the electron-poor diene (precursor) especially comprises a 5- or 6-ring (including optional hetero atoms).

Hence, in an embodiment the group T of the oxidizable precursor of the (oxo-group containing) (hetero)1,3-diene is selected from the group consisting of:

-C(R4)=C(R5)-;

-CHOH-;

-OCHOH-;

-S-;

and

-S(O)-,

wherein R4 and R5 are independently selected from the group consisting of H, OH and $NH_2$, and wherein especially at least one of R4 and R5 are selected from OH and $NH_2$.

When R4=OH and R5=H, the electron-poor diene may include a phenol, which may be converted to a diketon, e.g. with tyrosinase. When R4=R5=OH, the electron-poor diene may include benzenediol (ortho benzendiol or 1,2-catechol). Especially, at least one of R4 and R5 include OH.

Hence, in a further specific embodiment the oxidizable precursor of the (oxo-group containing) (hetero)1,3-diene is selected from the group consisting of a phenol, an *ortho*-benzenediol (1,2-catechol), an *ortho*-aminophenol (1,2-aminophenol), and an *ortho*-diaminobenzene (1,2- diaminobenzene), an *ortho*-dihydroxypyridine (2,3-dihydroxypyridine), an *ortho*-aminohydroxypyridine, an *ortho*-diaminopyridine. Hence, especially the electron-poor diene (precursor) comprises a 5 or 6 ring, with at least an OH or $NH_2$ group, especially two of such groups. As diketon groups in such dienes may be relatively unstable, the switchability of the system is of great use. A reaction partner with such diene

group can be provided without substantial stability problems. Shortly before the reaction has to take place, the precursor can be oxidized and the reaction can start. Oxidation can be executed before combining the reaction partners of interest, during combining the reaction partners of interest and after combining the reaction partners of interest. For instance, when the other system of interest is vulnerable to oxidation, oxidation of the precursor may be executed before combining the reaction partners. Hereby, a relative inert precursor is provided, whereas e.g. tetrazines are very reactive and cannot as easily be handled as the presently proposed precursor, whereas the subsequent chemical reaction can be as good (orthogonal, etc.), or even better, than with tetrazines.

Especially, the oxidizable precursor of the (oxo-group containing) (hetero)1,3-diene is selected from the group consisting of a phenol, an *ortho*-benzenediol, an *ortho*-aminophenol, an *ortho*-dihydroxypyridine, and an *ortho*-aminohydroxypyridine. In yet another embodiment, the oxidizable precursor of the (hetero)1,3-diene is selected from the group consisting of *ortho*-diaminobenzene and *ortho*-diaminopyridine.

As indicated above, oxidizing the oxidizable precursor of the (oxo-group containing) (hetero)1,3-diene to the oxo-group containing (hetero)1,3-diene defined by formula II, may be executed before combining, during combining or after combining, the reactive systems. Note that the invention does not exclude that oxidation is performed during two or more of those stages. Especially, the oxidaton is executed after combining the two reactive systems because - amongst others - a good mixing may then be obtained. Further, timing of the reaction can be well controlled.

In a specific embodiment, the oxo-group containing (hetero)1,3-diene defined by formula II is obtained in situ by oxidation using one or more of (i) an oxidizing reagent, (ii) an electrochemical oxidation, and (iii) an oxidizing enzyme. Alternatively or additionally, the oxo-group containing (hetero)1,3-diene defined by formula II is obtained in situ by catalytic oxidation. Catalytic oxidation may e.g. be executed with (transition) metal complexes, e.g. with one or more of Cu (II), Co(III), Mn (II), etc., in combination with e.g. molecular oxygen. Other oxidizing agents and/or catalytic agents may e.g. be selected from the group consisting of periodate, ceric ammonium nitrate, lead oxide, silver oxide, cobalt(II)salen (salen is a chelating ligand based on salicylaldehyde and ethylenediamine), benzodipteridine, diphenylcarbonyloxide, lithium perchlorate, $K_3Mo(CN)_8$, $K_3Fe(CN)_8$, peroxodisulfuric acid, sodium hypochlorite, iodine/sodium hydrogencarbonate, or other oxidizing reagents, especially in combination with air or molecular oxygen. Of course combinations of two or more different oxidizing reagents (including catalytic agents) may also be used. In yet a further specific embodiment, the periodate is selected from the group comprising sodium periodate, potassium periodate, sodium hydrogen periodate, and periodic acid.

Alternatively or additionally to the oxidizing agent and/or electrochemical oxidation, also an enzymatic oxidation may be applied, with an oxidizing enzyme. In a specific embodiment, the enzymatic conversion uses one or more enzymes selected from the group tyrosinase, catechol oxidase, polyphenol oxidase, catecholase, and catalase. Of course, also combinations of two or more different enzymes may be applied.

As further indicated above, P,Q,R,S of the electron-poor diene are independently selected from C or N. especially, all are C. Further, the electron-poor diene according to formula II may be substituted with one or more $R^{1'}$ groups (in the formula indicated with $(R^1)'$). The number of those substitutions is indicated with n'. The value of n' can be 16 or lower, such as 8 or lower. In general n' will 0-4, especially 0, 2 or 2, even more especially 0 or 1. $R^{1'}$ will further be elucidated below. Note that the group S in P,Q,R,S or the S in the P,Q,R,S ring is only used to indicate a group and does not indicate sulfur, as P,Q,R,S are independently selected from C and N. This is an exception for this ring, as elsewhere S indicates sulfur, unless indicated otherwise. Having discussed above the electron-poor diene, especially according to formula II, as well as its oxidation, now the cyclooctyne will be discussed in more detail.

The cyclooctyne may especially include a triple C-bond in a ring, with the ring comprising at least 8 atoms, and a second ring, in general smaller, having at least 3 atoms, of which two are shared with the other ring. The ring may optionally include heteroatoms. Referring to formula I, B and B' are independently selected from the group consisting of O, S, C(O), $NR^3$ and $C(R^3)_2$, wherein $R^3$ is independently selected from the group consisting of hydrogen, $R^1$ or $(L)_p$-$(A)_r$. Hence, B and B'in the formulas do not refer to boron, unless indicated otherwise.

The ring (with the triple bond) comprises $(CH2)_a$ and a $(CH2)_{a''}$ groups. The second ring includes a $(CH2)_{a'}$ group. The indices a, a' and a" are independently selected from 0 - 8, with the proviso that a + a' + a" = 4, 5, 6, 7 or 8.

Further, the cyclooctyne according to formula I may be substituted with one or more $R^1$ groups (in the formula indicated with $(R^1)$). The number of those substitutions is indicated with n. The value of n can be 16 or lower, such as 8 or lower. In general n will 0-4, especially 0, 2 or 2, even more especially 0 or 1.

Above, it has also been indicated that the electron-poor diene according to formula II can be substituted (with R').

The substitutions $R^1$ and $R^{1'}$ are independently selected from the group consisting of oxo, halogen, $-OR^2$, $-NO_2$, -CN, $-S(O)_2R^2$, $C_1$ - $C_{24}$ alkyl groups, $C_3$ - $C_{24}$ cycloalkyl groups, $C_2$ - $C_{24}$ (hetero)aryl groups, $C_3$ - $C_{24}$ alkyl(hetero)aryl groups and $C_3$ - $C_{24}$ (hetero)arylalkyl groups, wherein the alkyl groups, cycloalkyl groups, (hetero)aryl groups, alkyl(hetero)aryl groups and (hetero)arylalkyl groups are optionally substituted, wherein the alkyl groups, cycloalkyl groups, alkyl(hetero)aryl groups and (hetero)arylalkyl groups are optionally interrupted by one or more heteroatoms selected from the group consisting of O, S and N, and wherein $R^2$ is independently selected from the group consisting of hydrogen, halogen, $C_1$ - $C_{24}$ alkyl groups, $C_3$ - $C_{24}$ cycloalkyl groups, $C_2$-$C_{24}$ (hetero)aryl groups, $C_3$ - $C_{24}$ alkyl(hetero)aryl groups

and $C_3$ - $C_{24}$ (hetero)arylalkyl groups.

In a specific embodiment, when n is 2 or more, two $R^1$ groups may together form a (hetero)cycloalkyl group, the (hetero)cycloalkyl group optionally being substituted with an $(L)_p$-$(A)_r$ substituent. In yet another specific embodiment, when n is 2 or more, two $R^1$ groups may together form a (hetero)aryl group, the (hetero)aryl group optionally being substituted with an $(L)_p$-$(A)_r$ substituent. In yet a further specific embodiment, when n' is 2 or more, two $R^{1'}$ groups may together form a (hetero)cycloalkyl group, the (hetero)cycloalkyl group optionally being substituted with an $(L)'_p$-$(A)'_r$ substituent.

It is further noted that if q is 0, then B and/or B' is $NR^3$ wherein $R^3$ is $(L)_p$-$(A)_r$, and/or B and/or B' is $C(R^3)_2$ wherein one or more $R^3$ is $(L)_p$-$(A)_r$, and/or n is 2 or more and two $R^1$ groups together form a (hetero)cycloalkyl group wherein the (hetero)cycloalkyl group is substituted with an $(L)_p$-$(A)_r$ substituent, and/or n is 2 or more and two $R^1$ groups together form a (hetero)aryl group wherein the (hetero)aryl group is substituted with an $(L)_p$-$(A)_r$ substituent.

In a specific embodiment, the (hetero)cycloalkyne is selected from the group consisting of:

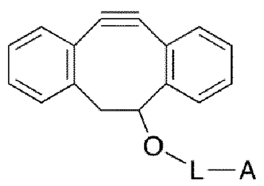
DIBO

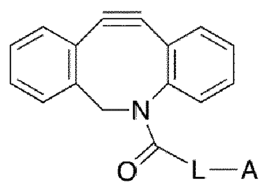
DIBAC / DBCO

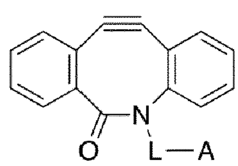
BARAC

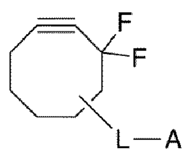
DIFO

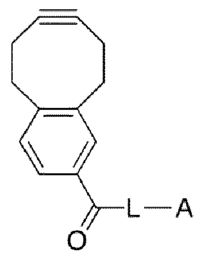
COMBO

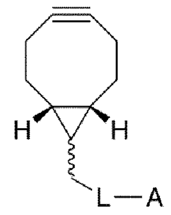
BCN

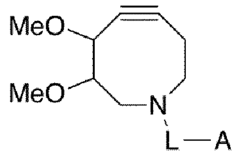
DIMAC

L-A is as defined in any one of the preceding claims.

Of course, combinations of two or more of these (hetero)cycloalkyne may also be applied. Further, other (hetero)cycloalkyne then depicted here may also be applied. In an embodiment, the first reaction partner comprises one or more of a non-benzoannulated cyclooctyne group, such as BCN or COMBO, etcl. In a specific embodiment, the first reaction partner comprises a bicyclononyne (BCN) group. Suitable cyclooctynes are e.g. described in US2013/0137763 and WO2014/065661, which are herein incorporated by reference.

In a specific embodiment, the first reaction partner comprises a cyclooctyne group and wherein the second reaction partner comprisies a 1,2-diol group, and wherein the method comprises (ii) oxidizing the 1,2-diol group to a 1,2-dione group, and (iii) allowing the first reaction partner with the cyclooctyne group and the second reaction partner with dione group to react with each other. For instnace, the first reaction partner comprises a bicyclononyne (BCN) group and wherein the second reaction partner comprises a 1,2-dione.

When the precursor of electron-poor diene according to formula II is obtained in its oxidized state, i.e. the electron-poor diene according to formula II, it will react with the cyclooctyne according to formula I. The cyclooctyne (according to formula I) and the electron-poor diene (according to formula II) may be allowed to react at e.g. room temperature, although elevated temperatures, such as up to 70 °C may also be possible. Further, the combination of the two reaction partners may especially be done while stirring or incubation.

Hence, in this way the invention provides the use of a (hetero)cycloalkyne and an oxo-group containing (hetero)1,3-diene for coupling a first system (A) of interest to a second system (A') of interest, wherein a (hetero)cycloalkyne is created in one of the first system (A) of interest and the second system (A') of interest, and wherein an oxo-group containing (hetero)1,3-diene is created in the other system of interest, and allowing the (hetero)cycloalkyne and the oxo-group containing (hetero)1,3-diene to react to form an A-A' conjugate. Especially, the oxo-group containing (hetero)1,3-diene is created in the other system of interest in a two stage process, wherein in a first stage an oxidizable precursor of the (oxo-group containing) (hetero)1,3-diene is created in the other system of interest and wherein in a second stage the oxidizable precursor of the (oxo-group containing) (hetero)1,3-diene is oxidized to the oxo-group containing (hetero)1,3-diene, followed by allowing the (hetero)cycloalkyne and the oxo-group containing (hetero)1,3-diene to react to form an A-A' conjugate, wherein the (hetero)cycloalkyne and the oxo-group containing (hetero)1,3-diene are as defined herein.

In this way, the conjugate defined by formula III is obtainable, wherein especially A and A' are independently selected from the group consisting of D, E and G, wherein D comprises a molecule of interest, E comprises a polymer or solid surface, and G comprises a functional group. In a specific embodiment of the conjugate Q,R,S,P,B,B' comprises C, wherein a + a' + a" = 3, especially wherein a + a' + a" = 3, wherein T comprises -C(O)C(O)-, wherein especially q,q' are 1, and wherein A and A' are selected from the combinations as defined herein (such as above). For instance, B and B' may be $C(R^3)_2$, wherein $R^3$ is hydrogen (i.e. B and B'are $CH_2$); Q,R,S,P may each be C. See for instance, Fig. 1, wherein such conjugate is depicted. Specific A-A' conjugates of interest may e.g. be selected from the combinations of a protein-label conjugate; a protein-protein conjugate; a protein-peptide conjugate; a protein- polymer conjugate; a protein-nanoparticle conjugate; a polymer network; a polymer hydrogel; and an antibody-drug conjugate.

The term "substantially" herein, such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of". The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The mere fact that certain measures are recited in mutually different dependent claims does not indicate that a combination of these measures cannot be used to advantage.

The invention further applies to a device comprising one or more of the characterizing features described in the description and/or shown in the attached drawings. The invention further pertains to a method or process comprising one or more of the characterising features described in the description and/or shown in the attached drawings.

The various aspects discussed in this patent can be combined in order to provide additional advantages. Furthermore, some of the features can form the basis for one or more divisional applications.

The compounds disclosed in this description and in the claims may comprise one or more asymmetric centers, and different diastereomers and/or enantiomers may exist of the compounds. The description of any compound in this description and in the claims is meant to include all diastereomers, and mixtures thereof, unless stated otherwise. In addition, the description of any compound in this description and in the claims is meant to include both the individual enantiomers, as well as any mixture, racemic or otherwise, of the enantiomers, unless stated otherwise. When the structure of a compound is depicted as a specific enantiomer, it is to be understood that the invention of the present application is not limited to that specific enantiomer.

The compounds may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer. The compounds disclosed in this description and in the claims may further exist as exo and endo diastereoisomers. Unless stated otherwise, the description of any compound in the description and in the claims is meant to include both the individual exo and the individual endo diastereoisomers of a compound, as well as mixtures thereof. When the structure of a compound is depicted as a specific endo or exo diastereomer, it is to be understood that the invention of the present application is not limited to that specific endo or exo diastereomer.

Furthermore, the compounds disclosed in this description and in the claims may exist as cis and trans isomers. Unless stated otherwise, the description of any compound in the description and in the claims is meant to include both the individual cis and the individual trans isomer of a compound, as well as mixtures thereof. As an example, when the structure of a compound is depicted as a cis isomer, it is to be understood that the corresponding trans isomer or mixtures of the cis and trans isomer are not excluded from the invention of the present application. When the structure of a compound is depicted as a specific cis or trans isomer, it is to be understood that the invention of the present application

is not limited to that specific cis or trans isomer.

The compounds disclosed in this description and in the claims may further exist as regioisomers. Unless stated otherwise, the description of any compound in the description and in the claims is meant to include both regioisomers of a compound, as well as mixtures thereof. When the structure of a compound is depicted as a specific regioisomer, it is to be understood that the invention of the present application is not limited to that specific regioisomer. Optionally, a + a' + a" is smaller than 4 or larger than 8.

EXPERIMENTAL

**General Method and Materials:**

Sulforhodamine B acid chloride, 4-*tert*-butyl-catechol and 2,2'-(ethylenedioxy)bis(ethylamine) were purchased from Sigma Aldrich and 3,4-dihyroxyphenylacetic acid and sodium periodate were purchased from Acros. All BCN-compounds and diazo-transfer reagent were purchased from SynAffix. *N*-(3-Aminopropanoyl)-dibenzoazacyclooctyne (DIBAC-$NH_2$) was a kind gift from SynAffix. Solvents were purchased from J.T. Baker, Sigma Aldrich or Fisher Scientific and used as received. Thin layer chromatography was performed on silica gel-coated plates (Kieselgel 60 F254, Merck, Germany) with the indicated solvent mixture, spots were detected by $KMnO_4$ staining (1.5 g $KMnO_4$, 10 g $K_2CO_3$, 2.5 mL 5% NaOH-solution, 150 mL $H_2O$) and UV-detection. NMR spectra were recorded on a Varian 400 (400 MHz) or an Advance III Bruker 500 (125 MHz) spectrometer. High-resolution mass spectra (HRMS) were recorded on a JEOL AccuTOF JMS-T100CS (Electrospray Ionization (ESI)) or a JEOL AccuTOF JMS-100GCv (Electron Ionization (EI), Chemical Ionization (CI)). The pEvol plasmid encoding the mutant pylRS/suppressor $tRNA_{CUA}$ and the plasmids encoding GFP were kindly provided by the laboratory of Dr. E.A. Lemke (EMBL, Heidelberg, Germany).

**Synthesis of 4-tert-butyl-1,2-benzoquinone (1):**

To a solution of 4-*tert*-butyl-catechol (1.0 g, 6 mmol) in 30 mL of THF/$H_2O$ (1:1) was added 6 mL of MeOH until the solution turned clear. Upon the portion wise addition of $NaIO_4$ (1.28 g, 6 mmol, 1 eq.) the solution turned into a dark reddish suspension, which was allowed to stir at room temperature for approximately 30 min. The reaction mixture was extracted with DCM (2 × 100 mL). The organic layers were combined, dried ($Na_2SO_4$), filtered and concentrated *in vacuo* to yield 1 as a brownish/reddish solid (0.98 g, 5.97 mmol, 99% yield). $R_F$ 0.35 (heptane/EtOAc, 2:1). $^1H$ NMR ($CDCl_3$, 400 MHz): $\delta$ = 7.22 (dd, J=10.4, 2.4 Hz, 1H), 6.40 (dd, *J*= 10.4, 0.8 Hz, 1H), 6.28 (dd, *J*= 2.4, 0.8 Hz, 1H), 1.25 (s, 9H). $^{13}C$ NMR ($CDCl_3$, 125 MHz): $\delta$ = 180.5, 180.4, 162.2, 140.2, 129.6, 124.0, 35.8, 27.9. HRMS (EI) for $C_{10}H_{12}O_2$: ($[M]^{+\bullet}$), calcd: 164.0837, found: 164.0839

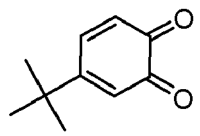

**Synthesis of compound 3:**

To a solution of 4-*tert*-butyl-1,2-benzoquinone (51 mg, 0.31 mmol) in MeOH/$H_2O$ (1:1) was added *endo*-bicyclo[6.1.0]non-4-yn-9-ylmethanol (*endo*-BCN, **2**) (70 mg, 0.46 mmol, 1.5 eq.). The reaction mixture was stirred at rt for 30 min. During the reaction the color changed from dark red to yellow. The reaction mixture was concentrated *in vacuo.* The residue was dry-loaded with silica and purified by column chromatography (1% triethylamine in EtOAc/pentane (1:2 → 1:1) to afford **3** as a yellow, glassy solid (77 mg, 0.24 mmol, 79% yield) as a mixture of *exo,endo* and *endo,endo* diastereoisomers **(3a** + **3b).** $R_F$ 0.10 (heptane/EtOAc, 2:1). $^1H$ NMR ($CDCl_3$, 400 MHz): $\delta$ = 6.08-6.05 (m, 2H, diastereoisomer a+b), 3.89-3.88 (m, 2H, diastereoisomer a + b), 3.73 (d, *J* = 6.4 Hz, 1H, diastereoisomer a), 3.73 (d, *J*= 6.4 Hz, 1H, diastereoisomer b), 3.69-3.67 (m, 4H, diastereoisomer a + b), 2.87-2.72 (m, 4H, diastereoisomer a + b), 2.30-2.21 (m, 4H, diastereoisomer a + b), 2.13-2.01 (m, 4H, diastereoisomer a + b), 1.80-1.68 (m, 4H, diastereoisomer a + b), 1.13 (s, 9H, diasteroisomer a), 1.12 (s, 9H, diasteroisomer b), 1.14-1.04 (m, 2H, diastereoisomer a + b), 0.89-0.68 (m, 4H). $^{13}C$ NMR ($CDCl_3$, 125 MHz): $\delta$ = 182.6, 182.5, 181.5, 181.3, 154.1, 153.8, 136.6, 136.4, 136.2, 135.7, 118.5, 118.4, 60.8, 60.4, 59.9, 59.9, 58.1, 58.0, 34.9, 34.9, 32.3, 32.2, 32.1, 31.64, 31.5, 28.3, 28.2, 22.7, 22.4, 22.3, 22.3, 20.2, 20.1, 17.9, 17.6, 17.3, 16.7. HRMS (CI) for $C_{20}H_{26}O_3$: (M + $H^+$-CO), calcd: 287.2006, found: 287.1996; (M+$CH_2CH_3^+$), calcd: 343.2273, found: 343.2290; (M + $CH_2CH_3^+$-CO) calcd: 315.2319, found: 315.2297.

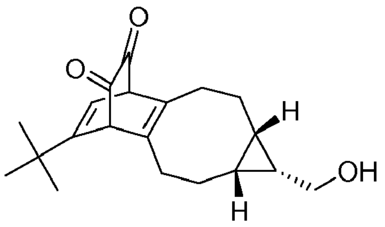

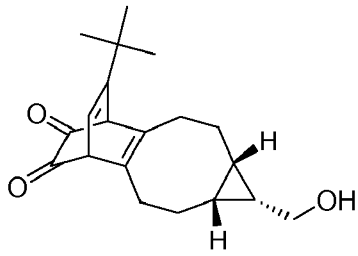

Retro-Diels-Alder products:

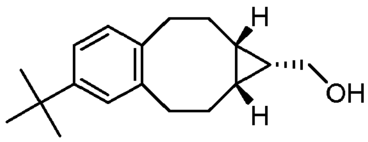

$^1H$ NMR ($CDCl_3$,400 MHz): δ = 7.14-7.11 (m, 2H), 7.04-7.02 (m, 1H). $^{13}C$ NMR ($CDCl_3$,125 MHz): δ = 148.8, 141.7, 139.1, 129.9, 127.2, 122.7. HRMS (CI) for $C_{18}H_{26}O$: (M + $H^+$-OH), calcd: 241.1956, found: 241.1936; (M + $H^+$-OH - t-Butyl), calcd: 185.1330, found: 185.1326.

**Synthesis of 3,4-O-isopropylidene-3,4-dihydroxy-phenylacetic acid 13:**

This compound was synthesized according to a previously published procedure [1] with an overall yield of 57%. $^1H$ NMR ($CDCl_3$, 400 MHz): δ = 6.68-6.67 (m, 3H), 3.54 (s, 1H), 1.66 (s, 6H). $^{13}C$ NMR ($CDCl_3$, 125 MHz): δ = 177.7, 147.8, 146.9, 126.2, 122.1, 118.2, 109.7, 108.3, 40.8, 26.0, HRMS (EI) for $C_{11}H_{12}O_4$: ($[M]^{+\bullet}$), calcd: 208.0736, found: 208.0741.

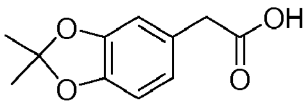

**General Peptide Synthesis:**

2-Chlorotrityl resin was purchased from Bachem, Fmoc-L-amino acids were purchased from Novabiochem or Bachem and coupling reagents were purchased from Biosolve and Sigma Aldrich. Lyophilization was achieved using an ilShin Freeze Dryer (ilShin, Ede, The Netherlands). Analytical HPLC was performed on a Shimadzu LC-20A Prominence system (Shimadzu) equipped with a C18 ReproSil column, 150 × 3 mm, particle size 3 μm (Screening Devices). Elution of the peptides was achieved using an $CH_3CN/H_2O$ gradient containing 0.1% TFA (5-100%, 1-30 min, flow 0.4 mL/min). LC-MS was performed on a Thermo Finnigan LCQ-Fleet ESI-ion trap (Thermo Fischer, Breda, The Netherlands) equipped with an Alltima C18 column (50 mm, 2 mm, particle size 3 μm) (Alltech Applied Sciences BV). An $CH_3CN/H_2O$ gradient containing 0.1% $HCO_2H$ was used for elution (5-100%, 1-10 min).

Peptides were synthesized from 1.0 g 2-chloro-trityl resin using Fmoc solid-phase peptide synthesis (SPPS). The resin was swollen in DCM for 20 min prior to use. Functionalization of the resin with the first amino acid was performed for 30 min with 2 eq. of the required amino acid and 3 eq. *N,N*-**diisopropylethylamine** (**DIPEA**). After the coupling reaction, capping of the resin was performed with 2 eq. DIPEA in MeOH. Deprotection of the Fmoc-groups was carried out with piperidine in DMF (20%, v/v) for 20 min. Subsequent couplings were performed for 45 min with 3 eq. of the required amino acid, 3.3 eq. of *N,N'*-diisopropylcarbodiimide (DIPCDI) and 3.6 eq. of *N*-hydroxybenzotriazole (HOBt) in DMF. After each coupling and deprotection step, a Kaiser test was done to ensure completion of the reaction. The peptides were cleaved from the resin by treatment with a mixture of TFA: triisopropylsilane (TIS):1,2-ethanedithiol (EDT):$H_2O$ (92.5: 2.5: 2.5: 2.5) for 3 h. Peptides were afforded by precipitation in $Et_2O$, followed by freeze-drying from water.

**Synthesis of peptide 4:**

The general procedure was followed for synthesis of the peptide. Compound **13** (2.5 eq.) was coupled as the

final residue for 4 h using DIPCDI (2.75 eq.) and HOBt (3.0 eq.) in DMF. The peptide was cleaved from the resin and obtained as a white solid.

LC-MS: m/z for $C_{38}H_{48}N_6O_{10}$: (M + $H^+$), calcd: 701.34, found: 701.3, Retention time = 6.43 min, analytical HPLC: Retention time = 12.58 min.

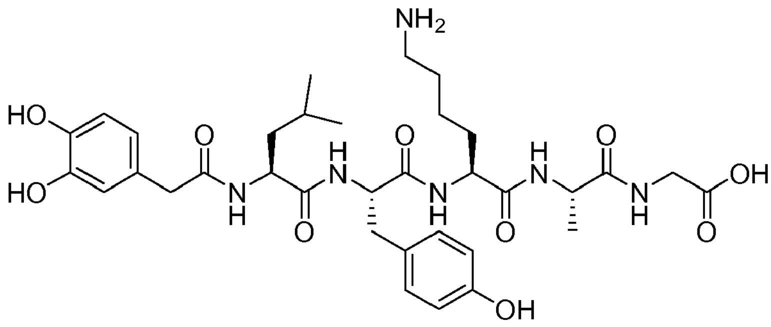

**Synthesis of peptide 7:**

Bromoacetic acid (13.9 g, 100 mmol) and sodium azide (13.0 g, 200 mmol, 2 eq.) were dissolved in water (60 mL) and stirred for 48 h at rt.[2] Concentrated hydrochloric acid (50 mL) was added to the reaction mixture after which extraction was performed with $Et_2O$ (4 × 100 mL). The combined organic phases were dried over $MgSO_4$ and evaporated in vacuo. The product was obtained as yellow oil and was dried under vacuum (9.86 g, 98%).

The general procedure was followed for synthesis of the peptide. As the final residue, azido acetic acid (5 eq.) from the previous step was coupled for 2 h using DIPCDI (5.5 eq.) and HOBt (6.0 eq.) in DMF. The peptide was cleaved from the resin and obtained as a white solid., LC-MS: m/z for $C_{28}H_{43}N_9O_8$: (M + $H^+$), calcd: 634.6, Retention time = 6.40 min, analytical HPLC: Retention time: 15.10 min.

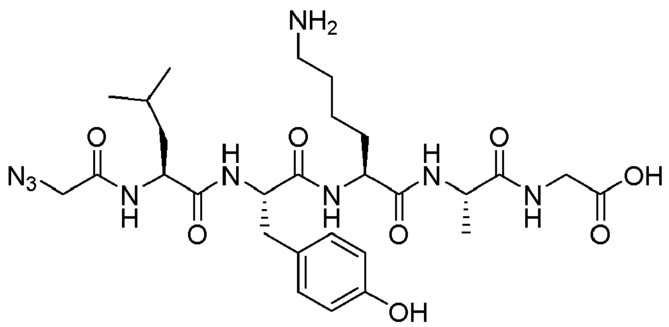

**Synthesis of catechol-functionalized dye 9:**

To a solution of **8** (50 mg, 0.070 mmol) in MeOH (4 mL) was added 10% Pd/C (10 mg) and $H_2$ was passed through for 4 h. The catalyst was filtered off over hyflo and the filtrate was concentrated *in vacuo.* The crude amine was dissolved in DMF (5 mL) and subsequently benzotriazol-1-yloxy-tris(dimethylamino)phosphonium hexafluorophosphate (BOP, Sigma Aldrich) (31 mg, 0.070 mmol), DIPEA (25 μL, 0.140 mmol), and 3,4-dihydroxyphenylacetic acid (12 mg, 0.070 mmol) were added. After stirring for 16 h at rt the mixture was concentrated *in vacuo.* The residue was purified by column chromatography ($H_2O$/ MeCN 1/20) to afford 9 (24 mg, 41%) as a dark red/purple solid. $R_f$ 0.37 ($H_2O$/ MeCN 1/12). $^1H$ NMR ($CDCl_3$ + $CD_3OD$, 400 MHz): δ 8.79 *(d, J* = 1.8 Hz, 1H), 8.1 (dd, *J* = 7.9, 1.9 Hz, 1H), 7.27 (d, *J* = 7.9 Hz, 1H), 7.15 (d, *J* = 7.9 Hz, 2H), 7.04 (t, *J* = 5.5 Hz, 1H), 6.77-6.68 (m, 6H), 6.58 (dd, *J* = 8.1, 2.1 Hz, 1H), 3.57-3.51 (m, 16H), 3.38-3.34 (m, 2H), 3.32 (s, 2H), 3.25 (t, *J* = 5.3 Hz, 2H), 1.29 (t, *J* = 7.1 Hz, 12H). $^{13}C$ NMR ($CD_3OD$, 125 MHz): δ 174.8, 159.4, 157.8, 157.1, 147.2, 146.4, 145.3, 144.2, 135.4, 133.7, 132.5, 129.3, 128.2, 127.7, 121.5, 117.3, 116.4, 115.3, 115.0, 97.0, 71.3, 71.3, 70.7, 70.5, 46.8, 44.1, 43.3, 40.5, 12.8. HRMS (ESI) m/z for $C_{41}H_{51}N_4O_{11}S_2$: (M + $H^+$), calcd: 839.2996, found: 839.2974.

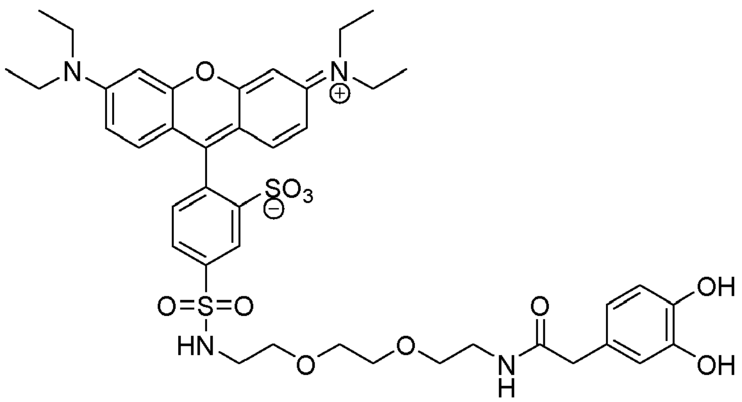

**Synthesis of azido-functionalized dye 11:**

To a solution of sulforhodamine B sulfonylchloride (364 mg, 0.631 mmol) in DMF (5 mL) was added at 0 °C DIPEA (169 $\mu$L, 0.947 mmol, 1.5 eq.) and *O*-(2-aminoethyl)-*O*'-(2-azidoethyl)ethylene glycol (110 mg, 0.631 mmol, 1eq.). After stirring for 16 h at rt the mixture was concentrated *in vacuo* and the residue was purified by column chromatography (column 1: MeOH/acetone/DCM, 1/4.5/4.5, column 2: MeOH/DCM, 1/9) which afforded 11 (180 mg, 40 %) as a dark red/purple solid. $R_f$ 0.21 (MeOH/DCM, 1/9). $^1$H NMR ($CDCl_3$, 400 MHz): $\delta$ 8.84 (d, *J*= 1.6 Hz, 1H), 7.98 (dd, *J* = 7.9, 1.9 Hz, 1H), 7.29 (d, *J* = 9.5 Hz, 2H), 7.21 (d, *J* = 7.9 Hz, 1H), 6.81 (dd, *J* = 9.5, 2.5 Hz, 2H), 6.67 (d, *J* = 2.5 Hz, 2H), 5.56 (t, *J*= 5.9 Hz, 1H), 3.75-3.72 (m, 2H), 3.71-3.64 (m, 6H), 3.61-3.50 (m, 8H), 3.47-3.45 (m, 2H), 3.30 (dd, *J*= 10.6, 5.6 Hz, 2H), 1.30 (t, *J* = 7.2 Hz, 12H). $^{13}$C NMR ($CD_3OD$ + $CDCl_3$, 125 MHz): $\delta$ 159.1, 157.7, 156.9, 147.1, 143.8, 135.1, 133.5, 132.1, 129.0, 127.6, 115.1, 114.8, 96.8, 71.3, 71.2, 70.9, 70.7, 51.5, 46.7, 43.9, 12.8. HRMS (ESI+) m/z for $C_{33}H_{43}N_6O_8S_2$: (M + $H^+$), calcd: 715.2584, found: 715.2571.

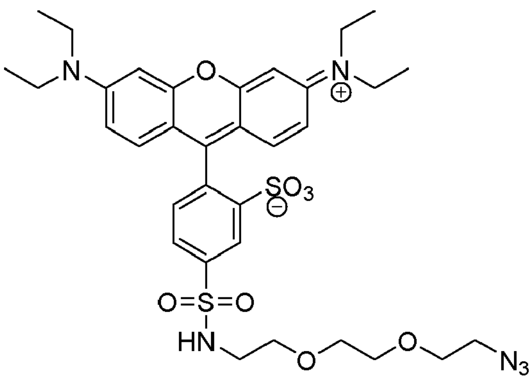

**Synthesis of bifunctional linker 12:**

To a solution of 3,4-dihydroxyphenylacetic acid (200 mg, 1.19 mmol) in DMF (2 mL) was added subsequently BOP (526 mg, 1.19 mmol), DIPEA (414 $\mu$L, 2.38 mmol) and *O*-(2-aminoethyl)-*O*'-(2-azidoethyl)ethylene glycol (249 mg, 1.43 mmol). After stirring for 16 h at rt the mixture was concentrated *in vacuo* and the residue was purified by column chromatography (column 1: MeOH/acetone/DCM/toluene, 0.1/2/8/1, column 2: MeOH/EA, 1/19) which afforded the product (281 mg, 73 %) as an oil. $R_f$ 0.32 (MeOH/EA, 1/19). $^1$H NMR ($CDCl_3$, 400 MHz): $\delta$ 6.76-6.72 (m, 2H), 6.58 (d, *J* = 7.9 Hz, 1H), 6.37 (t, *J* = 5.1 Hz, 1H), 3.64-3.57 (m, 6H), 3.52 (t, *J* = 5.0 Hz, 2H), 3.44-3.41 (m, 4H), 3.37-3.35 (m, 2H). $^{13}$C NMR ($CDCl_3$, 125 MHz): $\delta$ 173.3, 144.7, 144.0, 126.5, 121.4, 116.4, 115.8, 70.5, 70.2, 70.0, 69.6, 50.7, 42.8, 39.7. HRMS (ESI) m/z for $C_{14}H_{20}N_4NaO_5$: (M + $Na^+$), calcd: 347.1331, found: 347.1340.

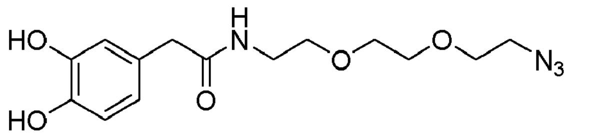

**Synthesis of *N*-(3-*N'*-acetyl-3-aminopropanoyl)-dibenzoazacyclooctyne (DIBAC-NHAc) (14):**

To a solution of DIBAC-$NH_2$ (80% purity, 90 mg, 0.33 mmol) in DCM (5 mL) was added $Ac_2O$ (46 $\mu$L, 0.49 mmol, 1.5 eq.), TEA (91 $\mu$L, 0.652 mmol, 2 eq.) and a cat. amount of 4-dimethylaminopyridine (DMAP). After stirring for 16 h at rt water (5 mL) was added and the mixture was extracted with DCM (3 $\times$ 10 mL). The organic layer was dried ($Na_2SO_4$), filtered and concentrated *in vacuo.* The residue was purified by column chromatography (MeOH/DCM, 1:19) to afford 14 (93 mg, 90%) as a white solid. $R_F$ 0.32 (MeOH/DCM, 1/19). $^1H$ NMR ($CDCl_3$, 400 MHz): $\delta$ 7.68 (d, J = 7.5 Hz, 1H), 7.42-7.26 (m, 7H), 6.07-6.02 (m, 1H), 5.14 (d, J = 13.9 Hz, 1H), 3.70 (d, J = 13.9 Hz, 1H), 3.38-3.30 (m, 1H), 3.24-3.16 (m, 1H), 2.46 (ddd, J = 16.6, 7.7, 4.0 Hz, 1H), 1.96 (ddd, J = 16.6, 7.3, 3.7 Hz, 1H), 1.81 (s, 3H). $^{13}C$ NMR ($CDCl_3$, 125 MHz): $\delta$ 172.5, 170.0, 151.2, 148.1, 132.2, 129.2, 128.7, 128.6, 128.5, 128.0, 127.4, 125.7, 123.1, 122.7, 114.9, 107.9, 55.7, 35.4, 34.9, 23.3. HRMS (ESI) m/z for $C_{20}H_{18}N_2NaO_2$: (M + $Na^+$), calcd: 341.1266, found: 341.1275.

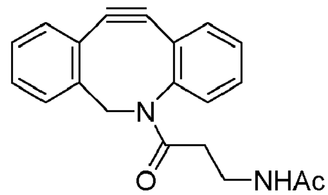

**Preparation of 15 by reaction of DIBAC-NHAc (14) and 1:**

To a solution of 14 (30 mg, 0.094 mmol) in DCM (2 mL) was added 1 (23 mg, 0.094 mmol, 1 eq.).The mixture was stirred for 3 h at rt in the dark. The solvent was evaporated and the residue was purified by column chromatography (MeOH/DCM, 1:19 $\rightarrow$1:9) to afford 15 (as a mixture of *endo*/*exo*-diastereomers and regioisomers, total 4 stereoisomers) (36 mg, 79%). $R_F$ 0.13-0.29 (MeOH/DCM, 19/1). $^1H$ NMR ($CDCl_3$, 400 MHz) and $^{13}C$ NMR ($CDCl_3$, 125 MHz) were recorded, but interpretation was impossible due to the overlap of signals for the 4 stereoisomers. $^1H$ and $^{13}C$ spectra are included. HRMS (ESI) m/z for $C_{30}H_{31}N_2O_4$: (M + $H^+$), calcd: 483.2284, found: 483.2290.

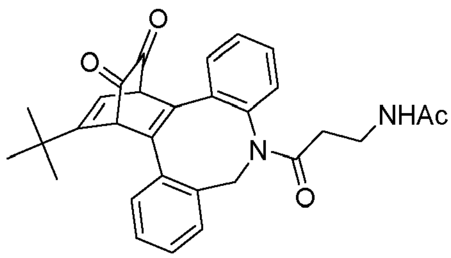

Retro-Diels-Alder products, mixture of regioisomers, HRMS (ESI) m/z for $C_{28}H_{31}N_2O_2$: (M + $H^+$), calcd: 427.2386, found: 427.2401.

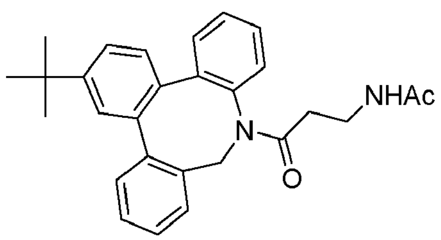

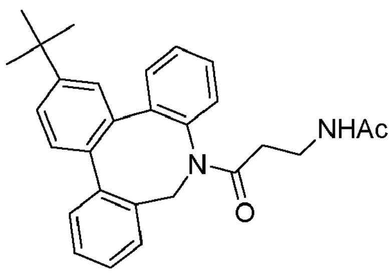

**Kinetic measurements:**

UV/Vis measurements were performed on a Varian Cary-50 spectrophotometer using a 1 cm quartz cuvette. A stock solution of BCN was prepared in MeOH/$H_2O$ (1:1) and filtered through an Acrodisc® 13 mm Syringe Filter (0.2 $\mu$m nylon membrane, Life Sciences). 4-*tert*-butyl-1,2-benzoquinone was also dissolved in MeOH/$H_2O$ (1:1) to a final concentration of 100 $\mu$M. For a total volume of 1 mL 20 $\mu$L of the BCN solution (final concentration 190 $\mu$M) was added

to the quartz cuvette followed by the addition of 980 μL of the 4-tert-butyl-1,2-benzoquinone solution. The reaction was followed at a wavelength of 384 nm with a scanning interval of 0.5 sec and was done *in triplo.* From the conversion plots thus obtained, second order rate constants were calculated according to this equation:

$$kt = \frac{1}{[B]_0 - [A]_0} \times \ln \frac{[A]_0 * ([B]_0 - P)}{[B]_0 * ([A]_0 - P)}$$

with k = $2^{nd}$ order rate constant ($M^{-1}s^{-1}$), *t* = reaction time (s), $[A]_0$ = the initial concentration of substrate A (mol/L), $[B]_0$ = the initial concentration of substrate B (mol/L) and [P] = the concentration of product (mol/L).

$^{1}H$ NMR monitoring of the cycloaddition of DIBAC-NHAc **14** with 1 was performed by rapid mixing (t=0) of stock solutions A and B (350 μL each) in an NMR tube and immediate insertion into a 400 MHz NMR spectrometer. NMR spectra were measured at preset time-intervals. The experiment was performed in triplo.

Stock solution A: **14** was dissolved in a mixture of $CD_3OD$ and $D_2O$ (ratio 1:1,1 mL) to give a 20.0 mM solution. Stock solution B: **1** was dissolved in a mixture of $CD_3OD$ and $D_2O$ (ratio 1:1,2 mL) to give a 24.0 mM solution. Kinetics of the reaction of **14** with **1** were determined by measuring the decrease of the integral of the signal at δ 3.72 caused by one DIBAC-NHAc methylene proton, compared to the integral of the $CD_3OD$ solvent-peak as internal standard. A starting value for the integral of the methylene signal was estimated, due to the fact that cycloaddition had already proceeded significantly by the time of the first measurement.

**HPLC analysis:**

Peptide stock solutions (10 mM) and a $NaIO_4$ stock solution (10 mM) were prepared in MilliQ, the BCN-biotin stock solution (10mM) was prepared in a mixture of acetonitrile and MilliQ (1:2). Peptide **4** and **7** were treated with sodium periodate (1 eq.) for 15 min at room temperature. Reactions were carried out in MilliQ with a final concentration of 750 μM peptide and 250 μM of BCN-biotin. The mixtures were incubated at room temperature for 1 h with moderate shaking and analyzed by LC-MS. 10 uL of the reaction mixtures were injected on a reverse-phase C18 column (50 mm, 2 mm, particle size 3 μm) and analyzed using a linear $CH_3CN/H_2O$ gradient with 0.5% $HCO_2H$ (5-100%, over 15 min). The corresponding mass spectra were acquired on a LCQ Fleet Ion Trap Mass Spectrometer (Thermo Scientific).

**Protein expression:**

TOP10 cells (Life technologies) were transformed with a pBAD plasmid carrying the sequence for an N-terminally FLAG-tagged and C-terminally His-tagged amber mutant green fluorescent protein (GFP) and a pEvol plasmid encoding for the $tRNA_{CUA}$/mutant-pylRS pair, that has been reported by *Plass et al.*[3] The protein expression was performed as described previously with minor changes.[4] Briefly, bacteria were grown in 2xYT medium with ampicillin and chloramphenicol at 37 °C until OD 0.4-0.6, when expression was induced with 0.02 % arabinose. BCN-lysine was dissolved in 0.1 M NaOH and added to the culture medium right before the induction with arabinose at a final concentration of 1 mM. Bacteria were grown for 16 h and harvested by centrifugation. The pellet was resuspended in 4x phosphate buffered saline (PBS, pH 8.0) with 10 mM imidazole and 1 mM phenylmethanesulfonyl fluoride (100 mM solution from Sigma Aldrich), and lysed by sonication. The lysate was centrifuged at 14.000xg at 4 °C for 25 min and the supernatant was incubated with Ni-NTA beads (Qiagen) for 2 h at 4 °C. Beads were washed and protein was eventually eluted using 4 × PBS, pH 8.0 and 500 mM imidazole. The buffer was exchanged for 1 × PBS, pH 7.4 using a HiPrep 26/10 desalting column (GE healthcare). The eluted fractions were combined and concentrated and stored at -20 °C in 15% glycerol until further usage.

**Protein labeling experiments:**

To determine the selectivity of SPOCQ, **9** (10 mM stock solution) was oxidized for 30 min at rt using 1 eq. of $NaIO_4$ (10 mM sock solution). 4 eq. of oxidized **9** was then incubated with $GFP^{BCN}$ (20 μM) and $GFP^{WT}$ (20 μM) at rt for 1 h with moderate shaking. Samples were then analyzed by SDS-polyacrylamide gel electrophoresis, in-gel fluorescence detection and coomassie staining.

To follow the labeling of $GFP^{BCN}$ as a function of time, **9** was oxidized as described earlier. For the control experiment, $NaIO_4$ was replaced by MilliQ. $GFP^{BCN}$ (final conc. 5 μM) was then incubated with 4 eq. of **9** (oxidized and not oxidized) and **11** and incubated at rt with moderate shaking. Samples were taken at indicated time points and were directly quenched with a 200-fold excess of BCN. Samples were eventually analyzed by SDS-polyacrylamide gel electrophoresis, in-gel fluorescence detection and coomassie staining.

**Protein dimerization experiment:**

Linker **12** was oxidized at rt for 30 min using $NaIO_4$ (1 eq.). $GFP^{BCN}$ (final concentration 50 μM) was incubated with the oxidized linker (5 eq.) for 1 h at rt, moderate shaking. The reaction mixture was transferred to a spin filter (10 K device, Amicon Ultra-0.5 mL, Millipore) and buffer was exchanged by the addition of 1 × PBS (3 × 15 min, at 4°C, 13000 × g). After spin-filtration, new $GFP^{BCN}$ was added (1 eq.) and incubated overnight at 37°C, moderate shaking. Next day, the reaction mixture was analyzed using size exclusion chromatrography (Superdex 75, Pharmaica), followed by SDS-polyacrylamide gel electrophoresis and coomassie staining. For the control experiment linker **9** was used without prior oxidation. $^1H$ NMR and $^{13}C$ NMR spectra of **15** were made. Kinetics of the cycloaddition of **14** with **1** using $^1H$ NMR, measurements were done *in triplo :* a decrease of the integral at δ 3.72 caused by one DIBAC-NHAc methylene proton during the reaction with **1** was observed. b) Determination of rate reaction constant for each measurement.

HPLC spectra and corresponding m/z spectra for the reaction of BCN-biotin (m/z = 551.6) with 3 eq. of peptide **4** (m/z = 701.4) with and without prior oxidation were measured. When peptide **4** was oxidized, a new peak (retention time: 9 min) with the expected mass of the anticipated product (m/z = 1250, hydrate: m/z = 1268) was detected. No reaction of BCN-biotin with peptide **4** was detected without prior oxidation. The peak with a retention time of 9.25 min in the right HPLC trace is an impurity from the peptide synthesis and can also be detected when only peptide **4** is injected.

HPLC spectra and corresponding m/z spectra for the reaction of BCN-biotin (m/z = 551.6) with 3 eq. of azido-functionalized peptide 7 (m/z = 634.6) in the presence of $NaIO_4$ were measured. The SPAAC reaction proceeded and two new peaks were detected with m/z values matching the anticpated products (m/z = 1201.0 (oxidation of biotin) and 1185.0 for peak 2 and 3, respectively).

HPLC spectra and corresponding m/z spectra for the reaction of BCN-biotin (m/z = 551.6) with 3 eq. of oxidized peptide **4** (m/z = 701.4) and azido-functionalized peptide 7 were also measured. A new peak (retention time: 9 min) with the expected mass of the anticipated product of the SPOCQ reaction was detected (m/z = 1250, hydrate: m/z = 1268), whereas the SPAAC product was not formed showing that SPOCQ outcompetes SPAAC due to its high reaction rate. $GFP^{BCN}$ and $GFP^{WT}$ were incubated with oxidized dye **9** for 1 h at rt. In-gel fluorescence analysis revealed that only $GFP^{BCN}$ has been fluorescently labeled showing the selectivity of the SPOCQ reaction.

A dimerization experiment with $GFP^{BCN}$ and linker **12** with and without prior oxidation analyzed by size exclusion chromatography was executed. The upper UV-chromatogram shows the presence of a new peak (elution time: 9 mL) that is absent in the control experiment (no oxidation of the linker). SDS-PAGE analysis of the corresponding fractions revealed that indeed the peak at 9 mL contained covalently formed $GFP^{BCN}$ dimer, whereas the peak with an elution time of 10 mL contained the GFP monomer.

**Experimental star-PEG-DOPA/star-PEG-BCN hydrogels**

*Star-PEG-BCN*

To a dry reaction flask was added 4-armed poly(ethylene glycol)-$NH_2$ HCl salt (600 mg, 0.06 mmol), (1*R*,8*S*,9*S*)-bicyclo[6.1.0]non-4-yn-9-ylmethyl succinimidyl carbonate (BCN-OSu; 87.4 mg, 0.30 mmol) and 100 μL triethylamine (100 μL, 0.72 mmol) in dry DCM (25 mL). The reaction mixture was stirred at r.t. overnight under $N_2$ atmosphere. Extraction was performed with 2M NaOH (3 x 25 mL). The organic layer was dried over $Na_2SO_4$ and concentrated in vacuo. The residue was purified by column chromatography on silica gel (MeOH:DCM 2:98, followed by 10:90). The product was afforded as a white powder after freeze-drying from dioxane (406 mg, 63%).

$R_f$= 0.36 (MeOH:DCM, 10:90); $^1H$-NMR (400 MHz, $CDCl_3$): δ = 0.86-1.01 (m, 4H), 1.25 - 1.43 (m, 8H), 1.52 - 1.66 (m, 8H), 2.18 - 2.34 (m, 16H), 3.38 (m, 16H), 3.51 - 3.86 (m, $[CH_2CH_2O]_n$), 4.15 (d, *J*= 8.0 Hz, 8H); MALDI-TOF (α-cyano-4-hydroxycinnamic acid): m/z: 10774 (calculated m/z: 10791 for 222 ethylene glycol units).

*Star-PEG-DOPA*

To star-poly(ethylene glycol)-$NH_2$ HCl salt (500 mg, 50 μmol) in DMF (13 mL) was added subsequently BOP (133 mg, 0.30 mmol); 3,4 dihydroxy phenylacetic acid (51 mg, 0.30 mmol), and D*i*PEA (210 μL, 1.2 mmol). The reaction mixture was stirred overnight at r.t., after which the DMF was evaporated in vacuo. The residue was dissolved in DCM and extraction was performed with 1 M $KHSO_4$ (3 x 40 mL) and brine (1 x 40 mL). The organic layer was dried over $Na_2SO_4$. Column chromatography on basic aluminum oxide (MeOH: DCM; 10:90) was utilized for further purification. Freeze-drying from dioxane afforded the product as a white powder (445 mg, 84%).

$R_f$= 0.25 (MeOH:DCM, 10:90); $^1H$-NMR (400 MHz, $CDCl_3$): δ = 3.36-3.86 (m, $[CH_2CH_2O]_n$, $C\underline{H}_2$-Ar-CH), 6.61 - 7.03 (m, Ar-CH) MALDI-TOF (α-cyano-4-hydroxycinnamic acid): m/z: 10913 (calculated m/z: 10909 for 227 ethylene

glycol units).

*Hydrogel formation*

Hydrogels were formed by addition of $NaIO_4$ or mushroom tyrosinase to an equimolair mixture of star-PEG-BCN and star-PEG-DOPA. Hydrogels were prepared in total polymer concentrations of 10, 20 and 30 mg/mL (1-3 wt%). Stock solutions of 20, 40 and 60 mg/mL of star-PEG-BCN and star-PEG-DOPA were first prepared in milliQ or PBS and then mixed in a 1:1 fashion. For hydrogels formed by sodium periodate, a stock solution containing 4 eq $NaIO_4$ (based on amount of star-PEG-DOPA, 1 eq $NaIO_4$ per DOPA group) was prepared in milliQ. Hydrogels formed by mushroom tyrosinase were formed in 1 x PBS (pH 7.5), which was bubbled through with $O_2$ for 15 min prior to use. A tyrosinase stock solution was prepared in PBS containing twice as much units/mL as requested in the final gel sample. For a typical hydrogel, equal volumes of the polymer mixture and the oxidizing agent ($NaIO_4$ or tyrosinase) were mixed to have the final concentration.

*Determination of gelation time*

Gelation time was determined qualitatively using the inverted vial test. The test was not suitable for hydrogels formed by sodium periodate, due to instant gel formation as soon as all components were mixed. For the enzymatically cross-linked hydrogels, the gelation times were determined using a tyrosinase concentration series: 100 - 2000 units/mL. For this, a 4000 units/mL stock solution in PBS was first prepared, of which a dilution series was made. Hydrogels (20 mg/mL) were formed using the typical procedure. As soon as the enzyme solution was added to the polymer mixture, the time measurement was started. When the material stopped flowing, and the vial could thus be inverted, the measurement was ended. Gelation time measurements were performed in triplo.

*Rheology*

The storage (G') and loss modulus (G") of the hydrogels were measured using an AR2000ex rheometer (TA instruments). All measurements were performed using a flat steel plate geometry (20 mm, gap size 500 $\mu$M) and were performed at a temperature of 20°C. Hydrogels were prepared by loading 100 $\mu$L of a star-PEG-BCN/star-PEG-DOPA mixture directly on the testing platform. After addition of a 100 $\mu$L $NaIO_4$ stock solution, the upper plate was immediately lowered and the measurement was started. Initially, a strain sweep measurement was conducted to determine the linear viscoelastic range of the hydrogels, by measuring between 0.1 and 1000% strain at an angular frequency of 10 rad/s. Within this range, a strain percentage (1%) was chosen to perform further measurements. The values of G' and G" were determined using an oscillatory time sweep test for 5 min at a constant strain of 1% and an angular frequency of 10 rad/s. For frequency sweep measurements, the angular frequency was measured between 0.1 and 100 rad/s and a constant strain of 1%. All measurements were performed in triplicate.

Hydrogel formation (20 mg/mL) was studied. With $NaIO_4$ and with mushroom tyrosinase gel formation occurred. However, without oxidizing agent (only star-PEG-DOPA and star-PEG-BCN) no gel formation was observed.

Gelation times of star-PEG-DOPA/star-PEG-BCN hydrogels formed by mushroom tyrosinase were measured. Hydrogels of 20 mg/mL were tested, formed by 100, 250, 500, 1000, 1500 and 2000 units/mL tyrosinase. Gelation was estimated to be fast, between 65 s ($\pm$ 8) for the 2000 units/mL and 1147 s($\pm$ 100) for the lowest enzyme concentration.

Rheological analysis was done on the gels formed with $NaIO_4$. Amongst others, representative time sweep measurements for a 20 mg/mL gel was performed. The gelation point (where G' exceeds G") could not be determined due to immediate gel formation. Stable values for G' and G" were obtained directly. The material shows typical hydrogel behaviour (G' >> G") and is highly elastic, with G' being about 512 Pa and with G" being about 16 Pa.

An overview of average G' values for 10, 20 and 30 mg/mL hydrogels (n = 5) was made. Stronger hydrogels are obtained with increasing polymer content (about 65 Pa, 454 Pa and 1375 Pa, respectively).

Also frequency sweep measurements for 10, 20 and 30 mg/mL hydrogels were performed. G' and G" values are independent of frequency, which corresponds to a predominantly elastic composition of the hydrogels.

Strain sweep measurement of a 20 mg/mL hydrogel to determine the linear viscoelastic region, with G' becoming lower than G" at about 1024% strain.

## Claims

**1.** A method for the oxidation-induced production of a conjugate defined by formula III from a first reaction partner comprising a (hetero)cycloalkyne defined by formula I and a second reaction partner comprising an oxo-group containing (hetero)1,3-diene defined by formula II according to the following scheme:

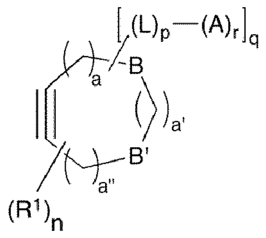

(I)

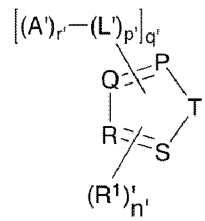

(II)

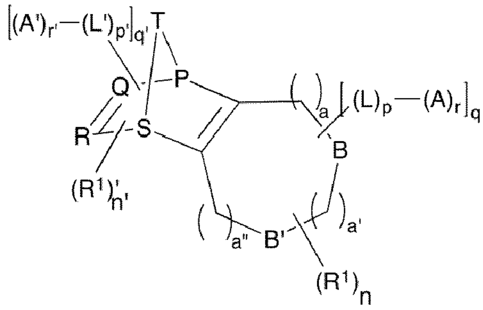

(III)

wherein the method comprises (i) combining the first reaction partner and the second reaction partner comprising an oxidizable precursor of the oxo-group containing (hetero)1,3-diene, (ii) oxidizing the oxidizable precursor of the oxo-group containing (hetero)1,3-diene to the oxo-group containing (hetero)1,3-diene defined by formula II, and (iii) allowing the (hetero)cycloalkyne and the oxo-group containing (hetero)1,3-diene to react to form the conjugate defined by formula III, wherein:

a, a' and a" are independently selected from 0 - 8, with the proviso that a + a' + a" = 4, 5, 6, 7 or 8;
n is 0-4; n' is 0-4
$R^1$ and $R^{1'}$ are independently selected from the group consisting of oxo, halogen, $-OR^2$, $-NO_2$, -CN, $-S(O)_2R^2$, $C_1$ - $C_{24}$ alkyl groups, $C_3$ - $C_{24}$ cycloalkyl groups, $C_2$ - $C_{24}$ (hetero)aryl groups, $C_3$ - $C_{24}$ alkyl(hetero)aryl groups and $C_3$ - $C_{24}$ (hetero)arylalkyl groups, wherein the alkyl groups, cycloalkyl groups, (hetero)aryl groups, alkyl(hetero)aryl groups and (hetero)arylalkyl groups are optionally substituted, wherein the alkyl groups, cycloalkyl groups, alkyl(hetero)aryl groups and (hetero)arylalkyl groups are optionally interrupted by one or more heteroatoms selected from the group consisting of O, S and N, and wherein $R^2$ is independently selected from the group consisting of hydrogen, halogen, $C_1$ - $C_{24}$ alkyl groups, $C_3$ - $C_{24}$ cycloalkyl groups, $C_2$ - $C_{24}$ (hetero)aryl groups, $C_3$ - $C_{24}$ alkyl(hetero)aryl groups and $C_3$ - $C_{24}$ (hetero)arylalkyl groups;
B and B' are independently selected from the group consisting of O, S, C(O), $NR^3$ and $C(R^3)_2$, wherein $R^3$ is independently selected from the group consisting of hydrogen, $R^1$ or $(L)_p$-$(A)_r$;

optionally, when n is 2 or more, two $R^1$ groups may together form a (hetero)cycloalkyl group, the (hetero)cycloalkyl group optionally being substituted with an $(L)_p$-$(A)_r$ substituent;
optionally, when n is 2 or more, two $R^1$ groups may together form a (hetero)aryl group, the (hetero)aryl group optionally being substituted with an $(L)_p$-$(A)_r$ substituent;
optionally, when n' is 2 or more, two $R^{1'}$ groups may together form a (hetero)cycloalkyl group, the (hetero)cycloalkyl group optionally being substituted with an $(L)'_p$-$(A)'_r$ substituent;

p is 0 or 1; p' is 0 or 1;
r is 1 - 4; r' is 1 - 4;
L is a linker; L' is a linker;
A is a first system of interest to be connected to a second system A' of interest;
q is 1 - 4; q' is 1 - 4;
P,Q,R,S are independently selected from C and N; and
T comprises an oxo-group selected from the group consisting of:

-C(O)C(O)-;

-C(O)C(=NR*), with R* being selected from the group consisting of H and alkyl and aryl group;

-C(O)-;

-OC(O)-;

-S(O)-;

and

-$SO_2$-.

2. The method according to claim 1, wherein A and A' are independently selected from the group consisting of D, E and G, wherein D comprises a molecule of interest, E comprises a polymer or solid surface, and G comprises a functional group.

3. The method according to any one of the preceding claims, wherein the group T of the oxidizable precursor of the oxo-group containing (hetero)1,3-diene is selected from the group consisting of:

-C(R4)=C(R5)-;

-CHOH-;

-OCHOH-;

-S-;

and

-S(O)-,

wherein R4 and R5 are independently selected from the group consisting of H, OH and $NH_2$, with at least one of R4 and R5 comprising OH or $NH_2$

4. The method according to any one of the preceding claims, wherein the (hetero)cycloalkyne is selected from the group consisting of:

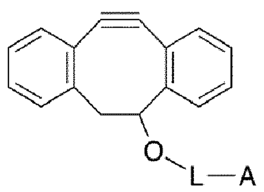
DIBO

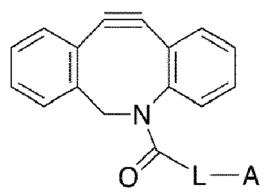
DIBAC / DBCO

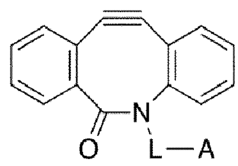
BARAC

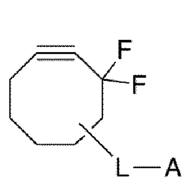
DIFO

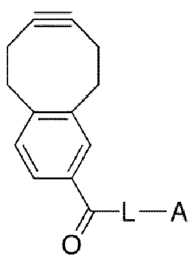
COMBO

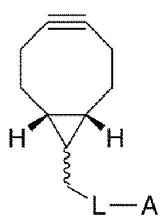
BCN

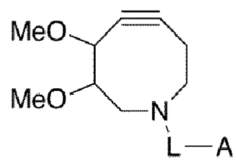
DIMAC

wherein L-A is as defined in any one of the preceding claims.

5. The method according to any one of the preceding claims, wherein the oxidizable precursor of the oxo-group

containing (hetero)1,3-diene is selected from the group consisting of a phenol, an *ortho*-benzenediol, an *ortho*-aminophenol, an *ortho*-dihydroxypyridine, and an *ortho*-aminohydroxypyridine.

**6.** The method according to any one of the preceding claims, wherein the first reaction partner comprises a cyclooctyne group and wherein the second reaction partner comprisies a 1,2-diol group, and wherein the method comprises (ii) oxidizing the 1,2-diol group to a 1,2-dione group, and (iii) allowing the first reaction partner with the cyclooctyne group and the second reaction partner with dione group to react with each other.

**7.** The method according to any one of the preceding claims, wherein the first reaction partner comprises one or more of a BCN group, a COMBO group or another non-benzoannulated cyclooctyne group.

**8.** The method according to any one of the preceding claims, wherein the oxo-group containing (hetero)1,3-diene defined by formula II is obtained in situ by oxidation using one or more of (i) an oxidizing reagent, (ii) an electrochemical oxidation, and (iii) an oxidizing enzyme.

**9.** The method according to any one of the preceding claims, the periodate is selected from the group comprising sodium periodate, potassium periodate, sodium hydrogen periodate, and periodic acid.

**10.** The method according to claim 9, wherein the enzymatic conversion uses one or more enzymes selected from the group tyrosinase, catechol oxidase, polyphenol oxidase, catecholase, and catalase.

**11.** The method according to any one of the preceding claims, wherein L and L' are independently selected from the group consisting of $C_1$-$C_{200}$ alkylene groups, $C_2$-$C_{200}$ alkenylene groups, $C_2$-$C_{200}$ alkynylene groups, $C_3$-$C_{200}$ cycloalkylene groups, $C_5$-$C_{200}$ cycloalkenylene groups, $C_8$-$C_{200}$ cycloalkynylene groups, $C_7$-$C_{200}$ alkylarylene groups, $C_7$-$C_{200}$ arylalkylene groups, $C_8$-$C_{200}$ arylalkenylene groups, and $C_9$-$C_{200}$ arylalkynylene groups, which may be substituted, unsubstituted, interrupted by one or more heteroatoms, branched or unbranched.

**12.** Use of a (hetero)cycloalkyne and an oxo-group containing (hetero)1,3-diene for coupling a first system (A) of interest to a second system (A') of interest, wherein a (hetero)cycloalkyne is created in one of the first system (A) of interest and the second system (A') of interest, and wherein an oxo-group containing (hetero)1,3-diene is created in the other system of interest, and allowing the (hetero)cycloalkyne and the oxo-group containing (hetero)1,3-diene to react to form an A-A' conjugate, especially wherein the oxo-group containing (hetero)1,3-diene is created in the other system of interest in a two stage process, wherein in a first stage an oxidizable precursor of the oxo-group containing (hetero)1,3-diene is created in the other system of interest and wherein in a second stage the oxidizable precursor of the oxo-group containing (hetero)1,3-diene is oxidized to the oxo-group containing (hetero)1,3-diene, followed by allowing the (hetero)cycloalkyne and the oxo-group containing (hetero)1,3-diene to react to form an A-A' conjugate, wherein the (hetero)cycloalkyne and the oxo-group containing (hetero)1,3-diene are as defined in any one of claims 1-10.

**13.** A conjugate defined by formula III

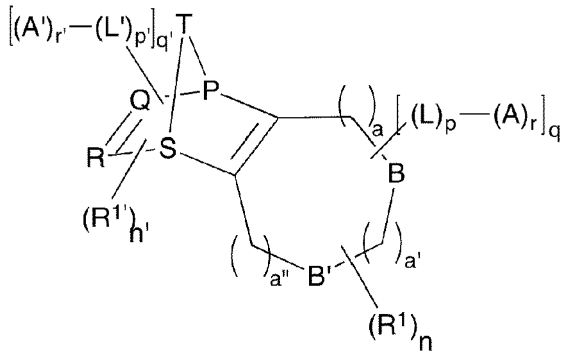

(III)

wherein A, A', L, L', p,p', $R^1$, $R^{1'}$, r, r', q,q',B,B', a, a', a", P, Q, R, S, T are as defined in any one of claims 1-11.

**14.** The conjugate according to claim 13, wherein A and A' are independently selected from the group consisting of D, E and G, wherein D comprises a molecule of interest, E comprises a polymer or solid surface, and G comprises a functional group.

**15.** The conjugate according to any one of claims 13-14, wherein Q,R,S,P,B,B'comprise C, wherein a + a' + a" = 4, wherein T comprises -C(O)C(O)-, wherein q,q' are 1, and wherein the A-A' conjugate is selected from the combinations of:

- a protein-label conjugate;
- a protein-protein conjugate;
- a protein-peptide conjugate;
- a protein- polymer conjugate;
- a protein-nanoparticle conjugate;
- a polymer network;
- a polymer hydrogel; and
- an antibody-drug conjugate.

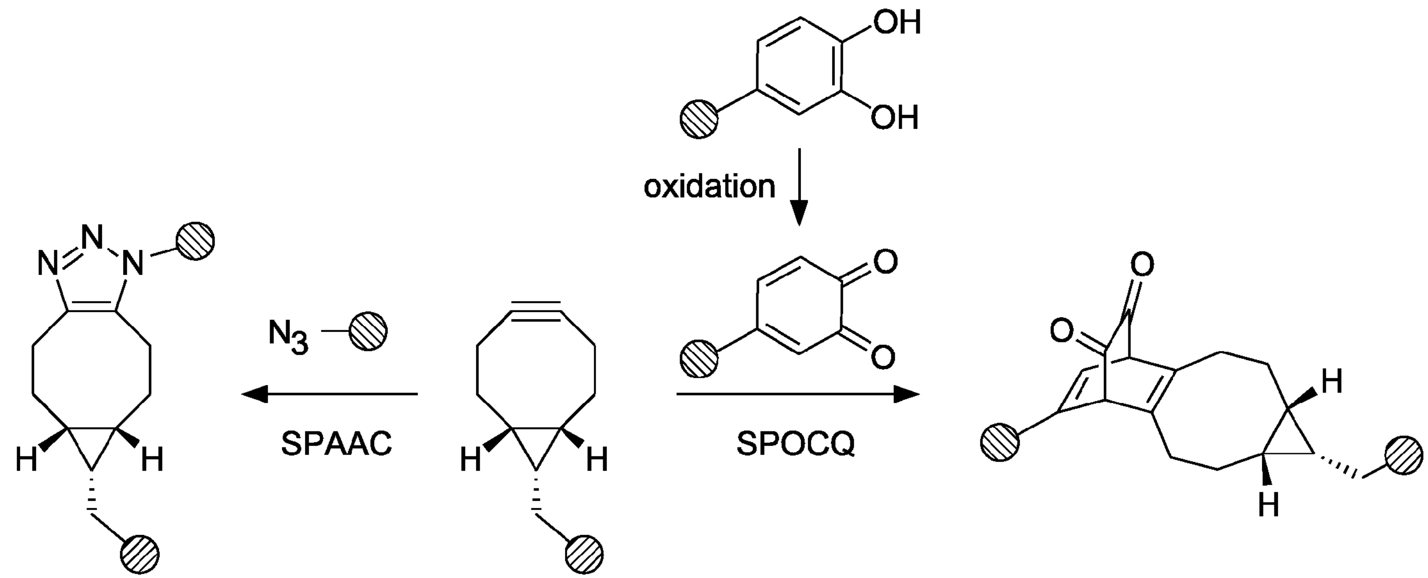


FIG. 1

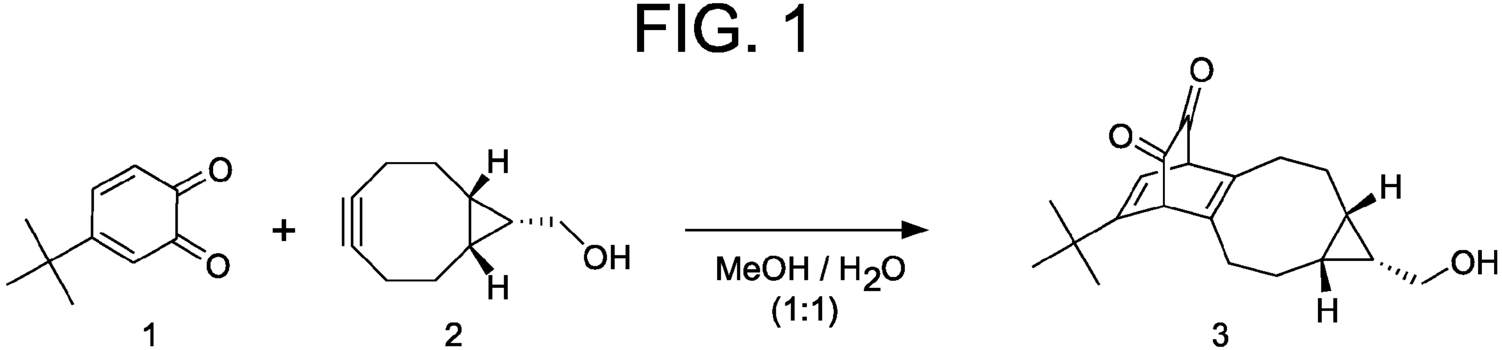


1 2 3

FIG. 2A

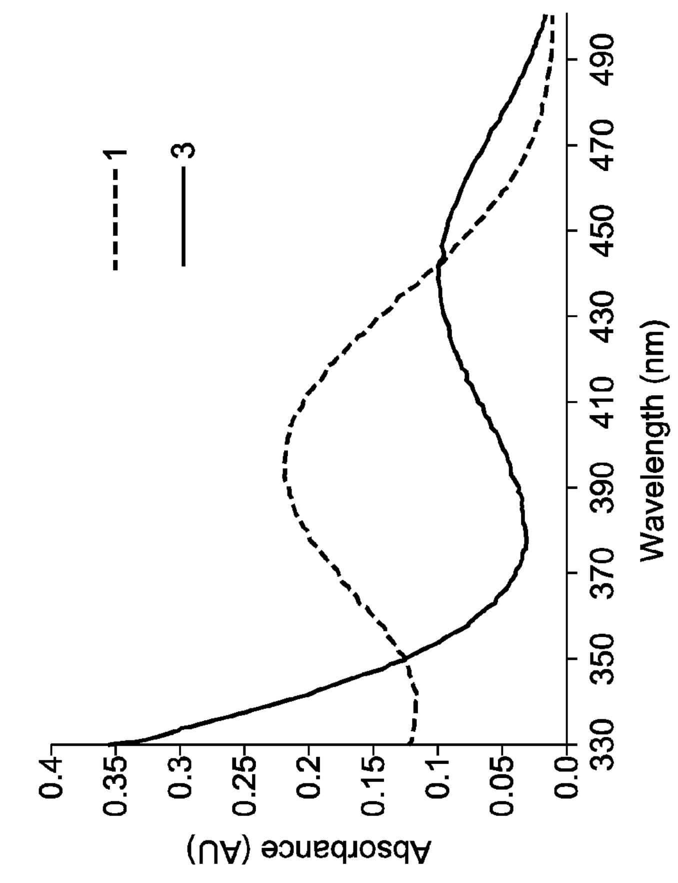

Absorbance (AU)
0.4
0.35
0.3
0.25
0.2
0.15
0.1
0.05
0.0
330
350
370
390
410
430
450
470
490
Wavelength (nm)
1
3


FIG. 2B

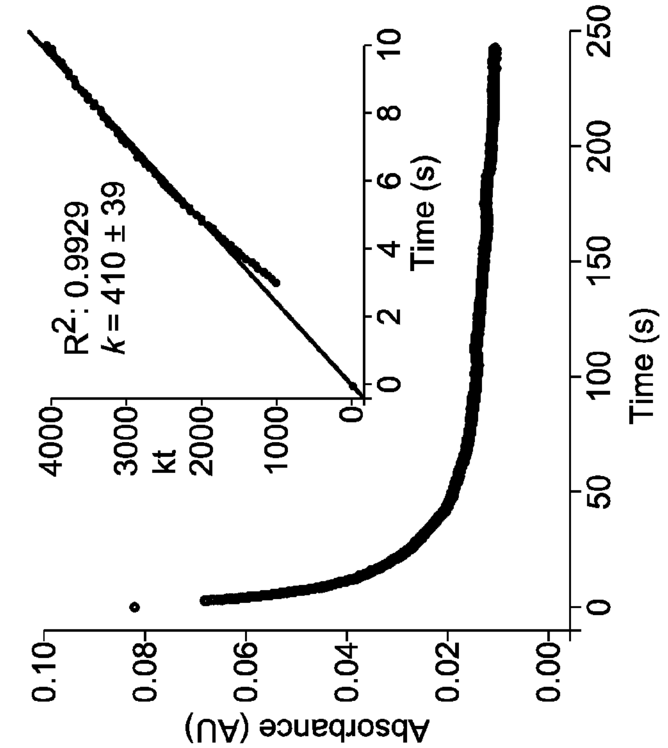

Absorbance (AU)
0.10
0.08
0.06
0.04
0.02
0.00
0
50
100
150
200
250
Time (s)
kt
4000
3000
2000
1000
0
0
2
4
6
8
10
Time (s)
$R^2$: 0.9929
$k$ = 410 ± 39


FIG. 2C

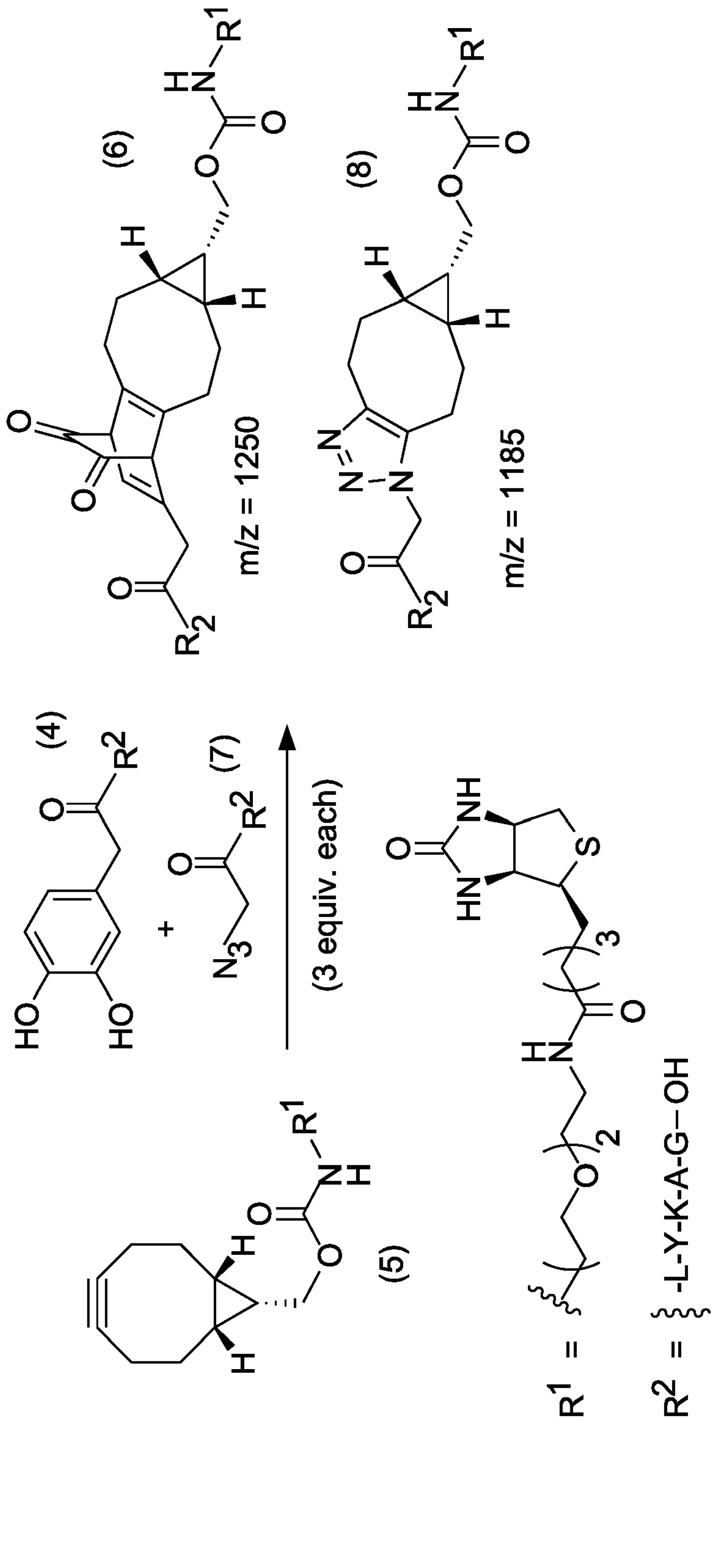


FIG. 3A

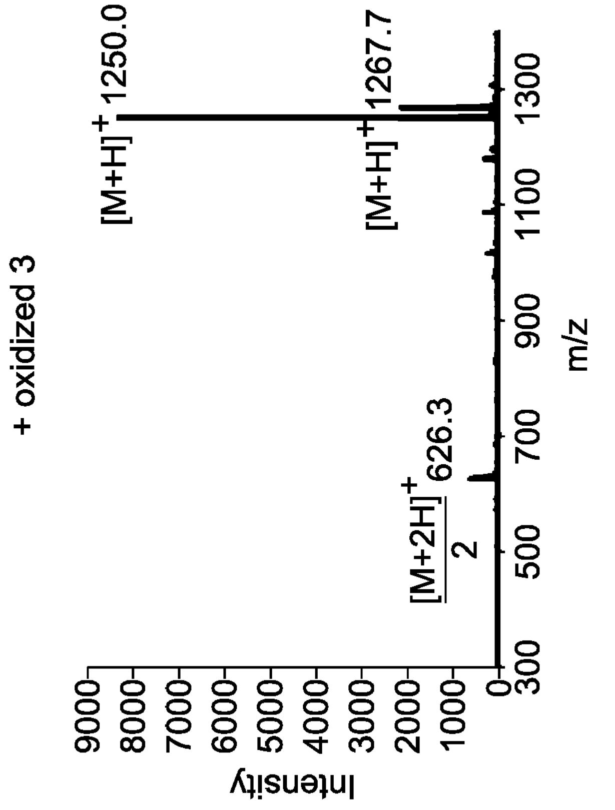
+ oxidized 3
Intensity
9000
8000
7000
6000
5000
4000
3000
2000
1000
0
300
500
700
900
1100
1300
m/z
[M+2H]+ / 2 626.3
[M+H]+ 1250.0
[M+H]+ 1267.7

FIG. 3B

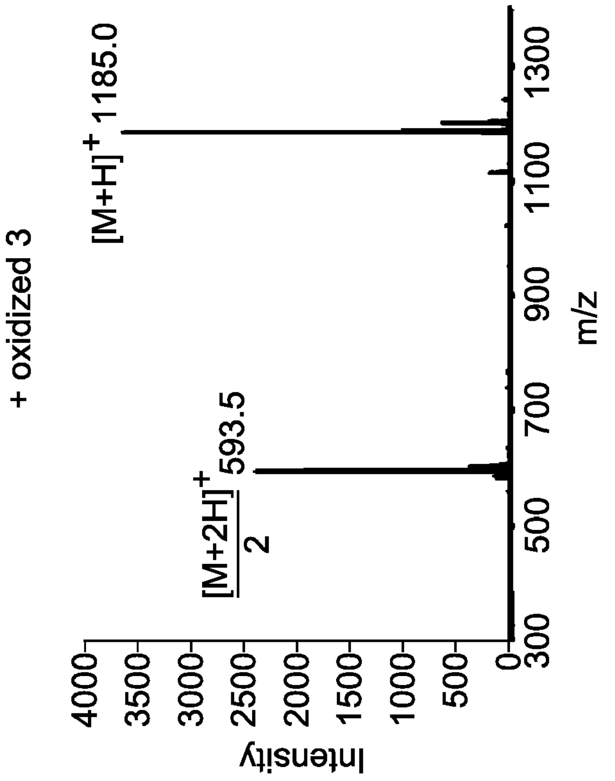
+ oxidized 3
Intensity
4000
3500
3000
2500
2000
1500
1000
500
0
300
500
700
900
1100
1300
m/z
[M+2H]+ / 2 593.5
[M+H]+ 1185.0

FIG. 3C

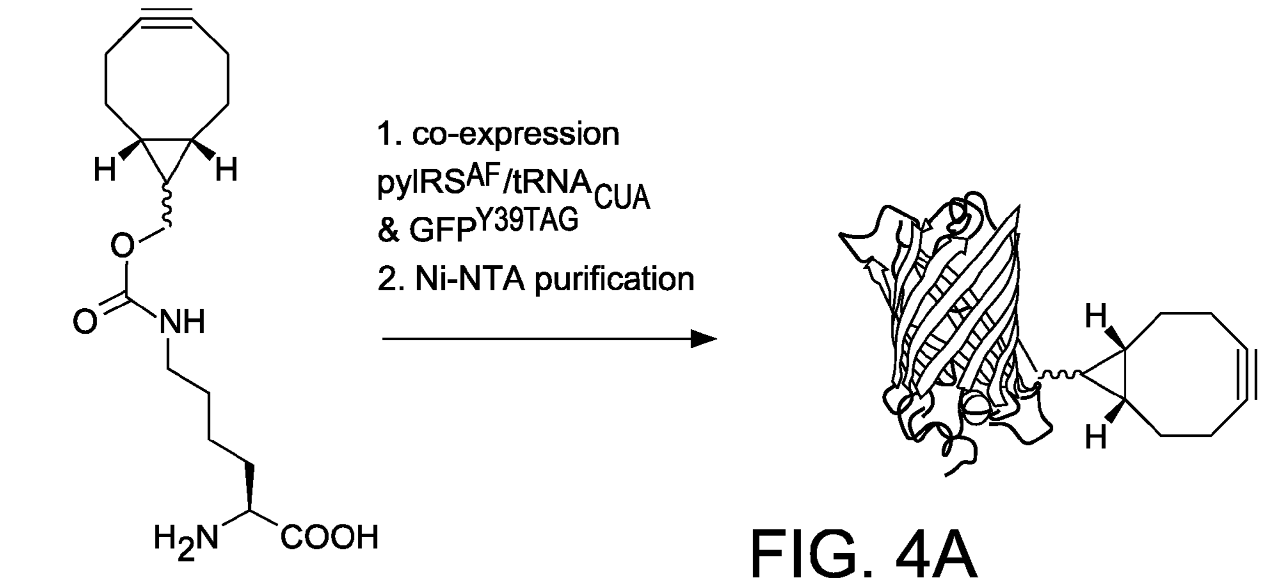


FIG. 4A

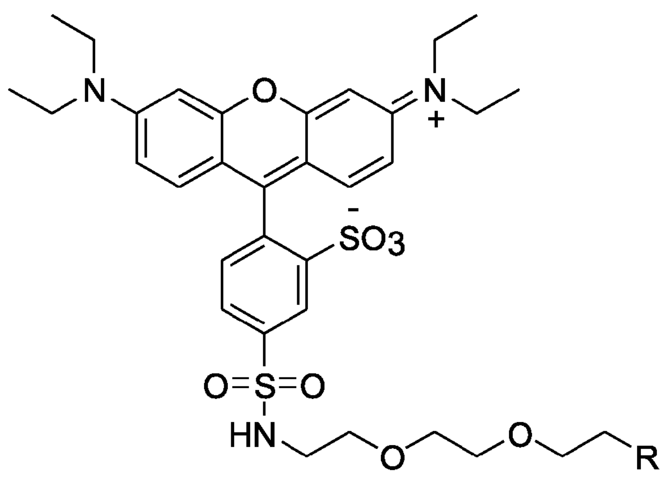

R =

9 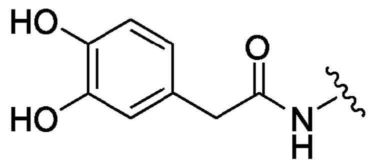

10 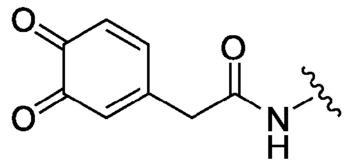

11 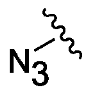

FIG. 4B

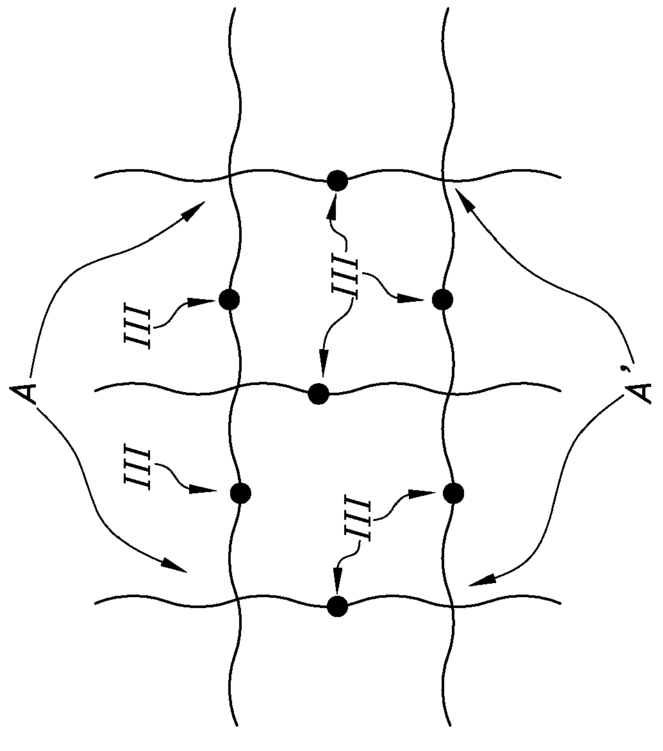


FIG. 5

Europäisches Patentamt
European Patent Office
Office européen des brevets

# EUROPEAN SEARCH REPORT

Application Number
EP 14 17 7230

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | P Molz ET AL: "Kinetische Untersuchungen zu Diels-Alder-Reaktionen des Cyclooctins mit anschlieaender Aromatisierung Kinetic Investigations of Diels-Alder-Reactions of Cyclooctyne with Consecutive Aromatization", Chem. Ber, 1 January 1984 (1984-01-01), pages 833-839, XP055160477, Retrieved from the Internet: URL:http://onlinelibrary.wiley.com/store/10.1002/cber.19841170234/asset/19841170234_ftp.pdf?v=1&t=i4nwap8i&s=a4ca7272bcb99bbeeadd223b07d86cc321ecfb37 [retrieved on 2015-01-08] * compounds 3a, 3b, 3e, 3f * ----- | 1-3,5-14 | INV. A61K47/48 A61K47/32 C07D225/08 |
| X | VERBOOM AND H J T BOS W: "Cycloaddition reactions of cyclooctyne with 9,10-phenanthrenequinone and 3,5-di-tert-butyl-o-benzoquinone", RECUEIL DES TRAVAUX CHIMIQUES DES PAYS-BAS, SOCIETE CHIMIQUE NEERLANDAISE, NL, vol. 100, no. 5, 1 January 1981 (1981-01-01), pages 207-213, XP008174027, ISSN: 0034-186X, DOI: 10.1002/RECL.19811000509 [retrieved on 2010-09-02] * scheme 4 * * compound 10 * ----- | 1-3,5-14 | TECHNICAL FIELDS SEARCHED (IPC) A61K C07D C07C |
| A,D | WO 2006/050262 A2 (UNIV CALIFORNIA [US]; BERTOZZI CAROLYN R [US]; AGARD NICHOLAS J [US];) 11 May 2006 (2006-05-11) * figure 1B * * examples 1-3 * ----- -/-- | 1-15 | |

1

The present search report has been drawn up for all claims

| Place of search | Date of completion of the search | Examiner |
|---|---|---|
| The Hague | 12 January 2015 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 14 17 7230

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2009/068738 A1 (BERTOZZI CAROLYN RUTH [US] ET AL) 12 March 2009 (2009-03-12)<br>* examples 1-6 *<br>* figure 1B *<br>----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 12 January 2015 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 17 7230

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2015

| Patent document cited in search report | Publication date | Patent family member(s) | Publication date |
|---|---|---|---|
| WO 2006050262 A2 | 11-05-2006 | EP 1812031 A2 | 01-08-2007 |
| | | US 2006110782 A1 | 25-05-2006 |
| | | US 2011213123 A1 | 01-09-2011 |
| | | WO 2006050262 A2 | 11-05-2006 |
| US 2009068738 A1 | 12-03-2009 | US 2009068738 A1 | 12-03-2009 |
| | | US 2013344527 A1 | 26-12-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006050262 A, Bertozzi
- WO 2014065661 A
- US 20130137763 A